# EUROPEAN PATENT SPECIFICATION

(11) **EP 3 512 882 B1**
(45) Date of publication and mention of the grant of the patent: **10.03.2021**
(21) Application number: 17769025.2
(22) Date of filing: 14.09.2017
(51) Int. Cl.: C07K 16/28, A61K 47/68, A61P 35/00

(54) **ANTI-C-MET ANTIBODIES AND ANTIBODY DRUG CONJUGATES THEREOF FOR EFFICIENT TUMOR INHIBITION**
ANTI-C-MET-ANTIKÖRPER UND ANTIKÖRPER-WIRKSTOFF-KONJUGATE DAVON ZUR EFFIZIENTEN TUMORHEMMUNG
ANTICORPS ANTI-C-MET ET CONJUGUÉS MÉDICAMENT-ANTICORPS DE CEUX-CI POUR UNE INHIBITION EFFICACE DES TUMEURS

(30) Priority: 14.09.2016 EP 16188857
(43) Date of publication of application: 24.07.2019
(73) Proprietor: Merck Patent GmbH, 64293 Darmstadt (DE)
(72) Inventor: DOERNER, Achim, 64293 Darmstadt (DE); TOLEIKIS, Lars, 67259 Kleinniedesheim (DE); PIATER, Birgit, 64285 Darmstadt (DE); RHIEL, Laura, 65843 Sulzbach (DE); KNUEHL, Christine, 64297 Darmstadt (DE); SELLMANN, Carolin, 64293 Darmstadt (DE); KRAH, Simon, 64283 Darmstadt (DE)
(86) International application number: PCT/EP2017/073106
(87) International publication number: WO 2018/050733

(56) References cited:
- WO-A1-2017/076492
- WO-A2-2007/110205
- WO-A2-2008/113013
- CRISTINA BASILICO ET AL: "Four individually druggable MET hotspots mediate HGF-driven tumor progression", JOURNAL OF CLINICAL INVESTIGATION, vol. 124, no. 7, 27 May 2014 (2014-05-27), pages 3172-3186, XP055425192, US ISSN: 0021-9738, DOI: 10.1172/JCI72316 cited in the application
- M. MERCHANT ET AL: "Monovalent antibody design and mechanism of action of onartuzumab, a MET antagonist with anti-tumor activity as a therapeutic agent", PROCEEDINGS NATIONAL ACADEMY OF SCIENCES PNAS, vol. 110, no. 32, 23 July 2013 (2013-07-23), pages E2987-E2996, XP55352711, US ISSN: 0027-8424, DOI: 10.1073/pnas.1302725110
- MARIA PRAT ET AL: "Monoclonal Antibodies against the MET/HGF Receptor and Its Ligand: Multitask Tools with Applications from Basic Research to Therapy", BIOMEDICINES, vol. 2, no. 4, 3 December 2014 (2014-12-03), pages 359-383, XP055425201, DOI: 10.3390/biomedicines2040359 cited in the application
- ELISA VIGNA ET AL: "Inhibition of ligand-independent constitutive activation of the Met oncogenic receptor by the engineered chemically-modified antibody DN30", MOLECULAR ONCOLOGY, vol. 9, no. 9, 5 June 2015 (2015-06-05), pages 1760-1772, XP055425203, AMSTERDAM, NL ISSN: 1574-7891, DOI: 10.1016/j.molonc.2015.05.007 cited in the application
- GREENALL SAMEER A ET AL: "Non-agonistic bivalent antibodies that promote c-MET degradation and inhibit tumor growth and others specific for tumor related c-MET", PLOS ONE, PUBLIC LIBRARY OF SCIENCE, US, [Online] vol. 7, no. 4, 1 April 2012 (2012-04-01), page e34658.1, XP002697775, ISSN: 1932-6203, DOI: 10.1371/JOURNAL.PONE.0034658 Retrieved from the Internet: URL:http://www.plosone.org/article/info%3A doi%2F10.1371%2Fjournal.pone.0034658> [retrieved on 2012-04-12]
- R CASTOLDI ET AL: "A novel bispecific EGFR/Met antibody blocks tumor-promoting phenotypic effects induced by resistance to EGFR inhibition and has potent antitumor activity", ONCOGENE, vol. 32, no. 50, 1 July 2013 (2013-07-01), pages 5593-5601, XP055129019, ISSN: 0950-9232, DOI: 10.1038/onc.2013.245
- LEE S. ROSEN ET AL: "A First-in-Human Phase I Study of a Bivalent MET Antibody, Emibetuzumab (LY2875358), as Monotherapy and in Combination with Erlotinib in Advanced Cancer", CLINICAL CANCER RESEARCH, vol. 23, no. 8, 10 October 2016 (2016-10-10), pages 1910-1919, XP55425244, US ISSN: 1078-0432, DOI: 10.1158/1078-0432.CCR-16-1418

## Description

### FIELD OF THE INVENTION

The present invention concerns anti-c-Met-specific antibodies and antibody drug conjugates thereof which are useful in the treatment of cancer. The present invention further provides method for producing the inventive antibody or antibody-drug conjugates thereof.

### BACKGROUND

The hepatocyte growth factor receptor (HGFR), also known as c-MET (for mesenchymal-epithelial transition), is a type I transmembrane protein RTK which was first identified as oncogenic TRP-MET fusion protein in chemically transformed human osteosarcoma cells. Expression of c-MET and secretion of its ligand, hepatocyte growth factor (HGF, or scatter factor, SF) by mesenchymal cells is involved in cell differentiation, proliferation, survival, cytoskeleton rearrangement, cell detachment, scattering, motility and invasiveness (Birchmeier et al (2003) Nat.Rev.Mol.Cell Biol. 4, 915-925). The proteoglycan decorin serves as a ligand for c-MET (Goldoni et al (2009) J.Cell Biol. 185, 743-754). On macro-cellular level, c-MET has diverse functions such as e.g. in embryogenesis, wound healing and organ regeneration. Tumorigenesis is characterized by morphological changes of cancer cells form epithelial to mesenchymal phenotype enabling metastatic cell spreading. In general, higher c-MET expression is found on metastatic lesions compared to primary tumor highlighting the involvement of c-MET in metastasis (Cipriani et al (2009) Lung Cancer 63, 169-179).

c-MET is a disulfide linked α-chain-β-chain heterodimer proteolytically cleaved from a single precursor protein. It is composed of a large extracellular domain composed of a seven-bladed propeller domain called SEMA, a PSI domain related to plexins, semaphorins and integrins as well as four IPT domain repeats displaying homology to immunoglobulins, plexins and transcription factor. The furin cleavage site between α- and β-chain is located between blade 4 and 5. The single spanning transmembrane domain is followed by an intracellular tyrosine kinase domain. Ligand binding to the receptor induces its dimerization and autophosphorylation of tyrosine residues 1230, 1235 and 1235 leading to transphosphorylation of tyrosines 1349 and 1356 which are docking sites for Src homology 2 proteins (SH2). (Birchmeier et al (2003) Nat.Rev.Mol.Cell Biol. 4, 915-925). Src recruitment subsequently activates intracellular signaling cascades including PI3K/AKT and Ras/MAPK pathways.

HGF is a disulfide linked α-chain-β-chain heterodimer which is processed from a single precursor protein (Lokker et al (1992) EMBO J. 11, 2503-2510). The HGF α-chain is composed of a N-terminal domain followed by four kringle domains, while the HGF β-chain consists only of one serine proteinase homology domain (SPH). HGF binds the proteoglycan heparin and forms HGF-homodimers in solution. For its interaction with c-MET a ligand-induced dimerization has been proposed in a 2:2 c-MET:HGF complex (Niemann (2013) Biochim.Biophys.Acta 1834, 2195-2204.; Stamos et al. (2004) EMBO J. 23, 2325-2335). There are two predicted binding sites for HGF on c-MET: The HGF β-chain is proposed to bind with low affinity to blades two and three of the SEMA domain (Gherardi et al. (2003) Proc.Natl.Acad.Sci.U.S.A 100, 12039-12044; Stamos et al. (2004) EMBO J. 23, 2325-2335), while there are two proposed binding sites for the high affinity binding of HGF's α-chain to c-MET. In a first model it has been proposed that IPT domains 3 and 4 of the shortened, N-terminal HGF fragment, called NK1, serves has the high affinity binding site, while in a scond proposed model the binding of NK1 to the SEMA domain blade 5 make up the high affinity binding site of HGF's α-chain to c-MET Youles et al. (2008) J.Mol.Biol. 377, 616-622).

Due to the involvement of c-MET in tumorigenesis and metastasis, several HGF and c-MET directed antibodies have been developed recently, some of which have been evaluated in clinical trials (Prat et al. (2014) Biomedicines 2, 359-383). HGF-neutralizing antibodies, such as e.g. rilotumumab and ficlatuzumab only target the ligand and are therefore inefficient in cancer with constitutive c-MET activation. Furthermore, HGF is stored in high abundance as an unprocessed precursor protein in the extracellular matrix of tissue hampering the efficiency of anti-HGF antibodies. Regarding c-MET, antibodies have been developed against several epitopes of the receptor: Onartuzumab (oa 5D5, MetMAb, RO5490258) and emibetuzumab (LY2875358) are the most advanced in clinical development with phase III and II trails, respectively. Even though the binding epitope of both antibodies is located within the c-MET SEMA domain, the monovalent onartuzumab predominantly acts via HGF-competition (Merchant et al. (2013) Proc.Natl.Acad.Sci.U.S.A 110, E2987-E2996) whereas the bivalent emibetuzumab induces receptor degradation besides the blockade of ligand binding (Liu et al. (2014) Clin.Cancer Res. 20, 6059-6070.). Three additional anti-c-MET antibodies are currently evaluated in clinical phase I: the bivalentABT-700 (h224G11, the ADDC-enhanced ARGX-111 (WT52-E) (Basilico et al. (2014) J.Clin.Invest 124, 3172-3186; Hultberg et al. (2015) Cancer Res. 75, 3373-3383), and the bivalent c-MET degrading antibody SAIT301 (Oh et al. (2012) Mol.Cells 34, 523-529.). While ARGX-111 and emibetuzumab occupy an overlapping epitope, the exact binding site of SAIT301 is unknown. The latter induces c-MET degradation via LRIG-mediated lysosomal pathway. (Lee et al. (2014) Oncogene 33, 34-43). This is presumably the same mode of action as for LMH 87 binding to the top of the SEMA propeller on blade 3 and 4. (Greenall et al. (2012) PLoS.One. 7, e34658; Prat et al. (2014) Biomedicines 2, 359-383). However, SAIT301 and LMH 87 do not share an overlapping epitope. Another c-MET degrading antibody is DN30 which binds to the forth IPT domain, induces degradation via the metalloprotease ADAM-10 and does not compete with ligand binding. (Vigna et al. (2015) Mol. Oncol. 9, 1760-1772). Some bivalent anti-c-MET antibodies are prone to trigger partial or full agonism requiring monovalent formats in parts for therapeutic application. Adverse events of the c-MET targeting antibody, e.g. onartuzumab, are mainly edema and thrombotic events being associated with the blockade of the c-MET/HGF axis regulating epithelial integrity and wound healing.

There is thus a continuous need to expand the repertoire of high affinity anti-c-MET antibodies and corresponding antibody-drug conjgutes for use in the treatment of cancer which overcome the limitations of anti-c-MET antibodies available in prior art.

### SUMMARY OF THE INVENTION

The present inventors have surprisingly found anti-c-Met antibodies or antigen-binding fragments thereof, which bind c-MET with high affinity and may be used to efficiently inhibit c-MET expressing tumors.

The present invention provides anti-c-MET antibodies or antigen-binding fragments thereof, which bind to human c-MET with an affinity of at least 10⁻⁸ M.

According to one embodiment the inventive antibodies or antigen-binding fragments as disclosed above bind to human c-MET variant N375S.

In one embodiment the inventive antibodies or antigen-binding fragments therof as disclosed above bind to an epitope comprised in the SEMA domain of human c-MET and inhibits c-MET signaling.

According to one embodiment the inventive antibody or antigen-binding fragment as disclosed above bind to an epitope comprised in IPT domains 1-4 of human c-MET and inhibits c-MET signaling.

In one embodiment the inventive antibodies or antigen-binding fragments thereof as disclosed above inhibit the binding of recombinant human HGF to human c-MET ECD at a concemtration of 0.88x10⁻⁹M or less by 50% in an enzyme-linked immunosorbent assay using HGF in solid phase.

According to one embodiment the antigen-binding fragments of the inventive antibody is a Fab, a F(ab')₂, or a scFv.

The inventive antibody is an IgG type antibody.

The inventive antbodies as disclosed above comprise heavy and light chain amino acid sequences according to SEQ ID NO:1 and SEQ ID NO: 2, or SEQ ID NO: 3 and SEQ ID NO: 4, or SEQ ID NO: 5 and SEQ ID NO: 6.

In one embodiment the inventive antibodies or antigen-binding fragments thereof are further coupled to a diagnostic or therapeutic agent.

In one embodiment the present invention provides a heterodimeric immunoglobulin molecule comprising
(i) a first and/or second Fab or scFv fragment which specifically bind(s) to human c-MET, and
(ii) an antibody hinge region, an antibody CH2 domain and an antibody CH3 domain comprising a hybrid protein-protein interaction interface domain wherein said interaction interface domain is formed by amino acid segments of the CH3 domain of a first member and amino acid segments of the CH3 domain of said second member, wherein said protein-protein interface domain of the first chain is interacting with the protein-protein-interface of the second chain by homodimerization of the corresponding amino acid segments of the same member of the immunoglobulin superfamily within said interaction domains,
wherein the first engineered immunoglobulin chain has the polypeptide sequence ("AG-SEED"):
GQPFRPEVHLLPPSREEMTKNQVSLTCLARGFYPKDIAVEWESNGQPENNYKTTPS RQEPSQGTT TFAVTSKLTVDKSRWQQGNVFSCSVMHEALHNHYTQKTISL and the second engineered immunoglobulin chain has the polypeptide sequence ("GA-SEED"): GQPREPQVYTLPPPSEELALNELVTLTCLVKGFYPSDIAVEWLQGSQELPREKYLT WAPVLDSDG SFFLYSILRVAAEDWKKGDTFSCSVMHEALHNHYTQKSLDR and wherein the first and/or second Fab or scFv fragment comprise at least two of the amino acid sequences according to SEQ ID NO: 1, SEQ IDNO: 2, SEQ ID NO: 3, SEQ ID NO: 4, SEQ ID NO: 5, SEQ ID NO: 6, SEQ ID NO: 7, SEQ ID NO: 8.

In one embodiment the inventive heterodimeric immunoglobulin molecule is further coupled to a diagnostic or therapeutic agent.

According to one embodiment the heterodimeric immunoglobulin molecule according to the invention is coupled to a cytotoxin.

In one embodiment the heterodimeric immunoglobulin molecule of the invention is afucosylated.

In one embodiment the inventive antibody, or the heterdimeric immunoglobulin molecule as disclosed above may be for use in the treatment of cancer.

### BRIEF DESCRIPTON OF DRAWINGS

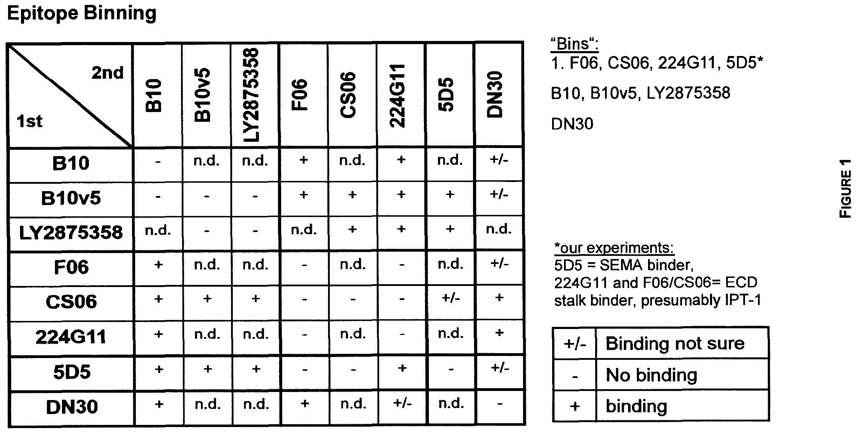

- **Figure 1:**: Epitope binning of the inventive antibodies or heterodimeric immunoglobulin molecules

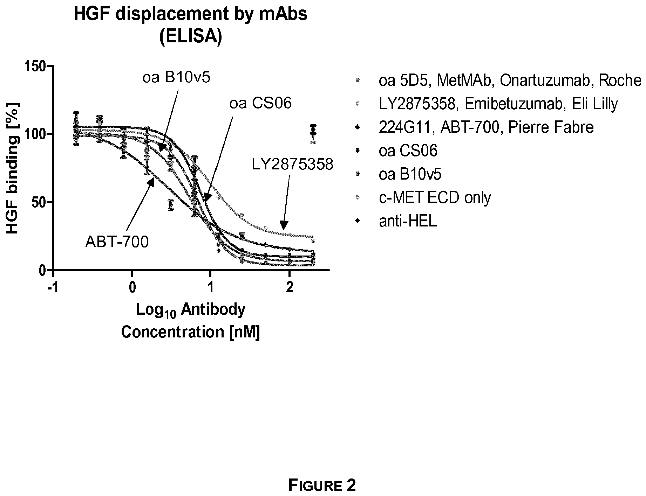

- **Figure 2:**: HGF displacement ELISA results

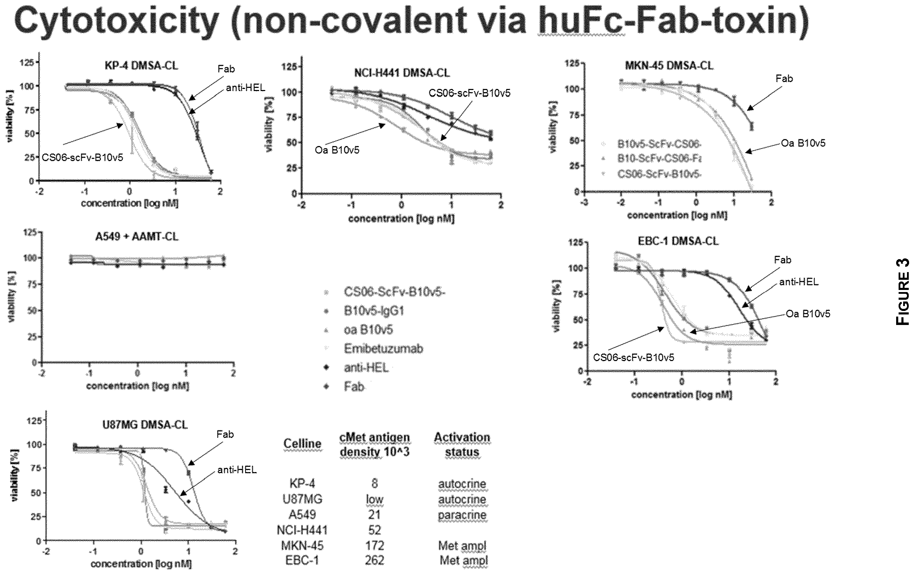

- **Figure 3:**: Cytotoxicity assay for antibody drug conjugates using non-covelantly coupled to the cytotoxin duocarmycin SA (DMSA). The results indicate cytotoxic effects dependent on c-MET expression by the respective cell lines tested.

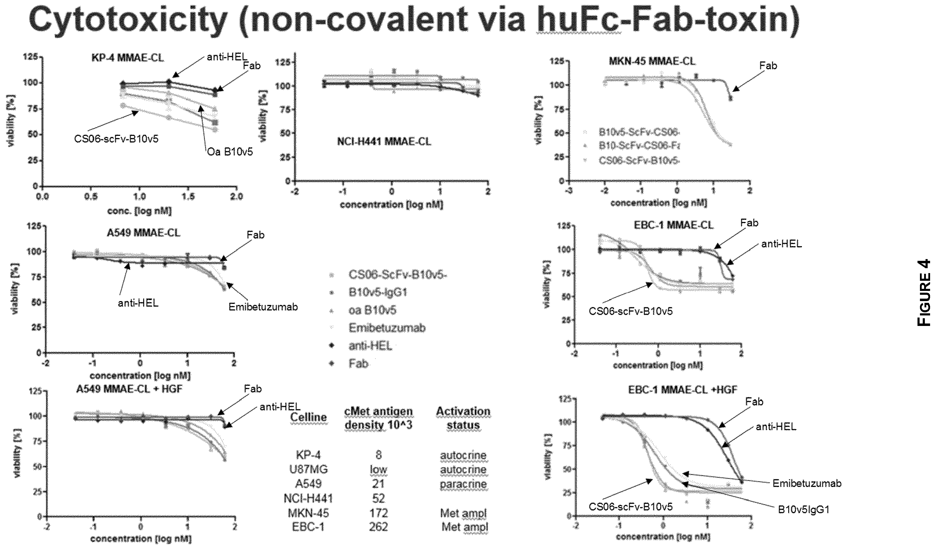

- **Figure 4:**: Cytotoxicity assay for antibody drug conjugates using non-covelantly coupled to the cytotoxin monomethyl auristatin E (MMAE). The results indicate cytotoxic effects dependent on c-MET expression by the respective cell lines tested
- **Figure 5:**: Inhibition of c-MET signaling by inventive antibodies as assessed by a c-MET-specific phosphorylation assay. Inhibition was assessed at a concentration of 167nM of the inventive antibodies or heterodimeric immunoglobulin molecules as indicated. MetMab® and emibetuzumab represent controls.

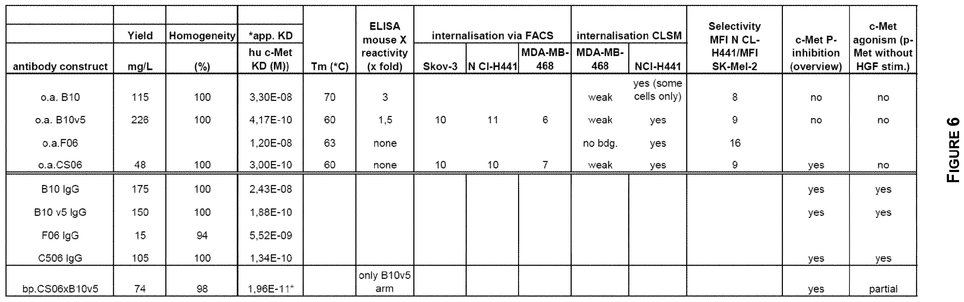

- **Figure 6:**: Summary of antibody properties. Approx. KDs of inventive antibodies to human c-MET ECD (oa CS06: 3x10⁻¹⁰M, oa B10v5: 4.17x10⁻¹⁰M, B10v5 IgG1: 1.88x10⁻¹⁰M, CS06 IgG1: 1.34x10⁻¹⁰M). Inventive biparatopic bispecific heterodimeric immunoglobulin molecule comprising Fab fragments CS06 and B10v5 ("bp CS06xB10v5": 1.96x10⁻¹¹M).

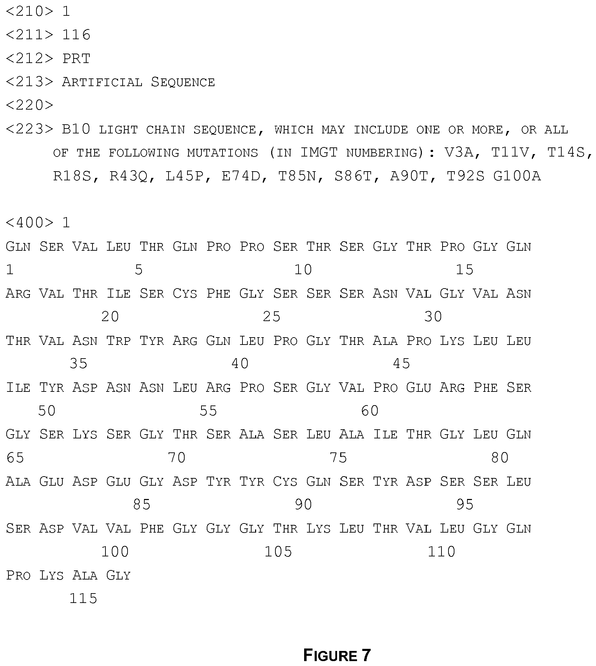

- **Figure 7:**: SEQ ID NO: 1

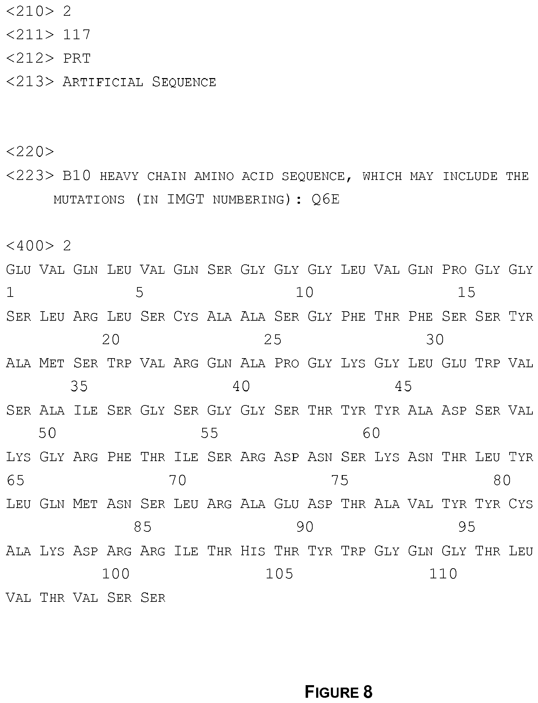

- **Figure 8:**: SEQ ID NO: 2

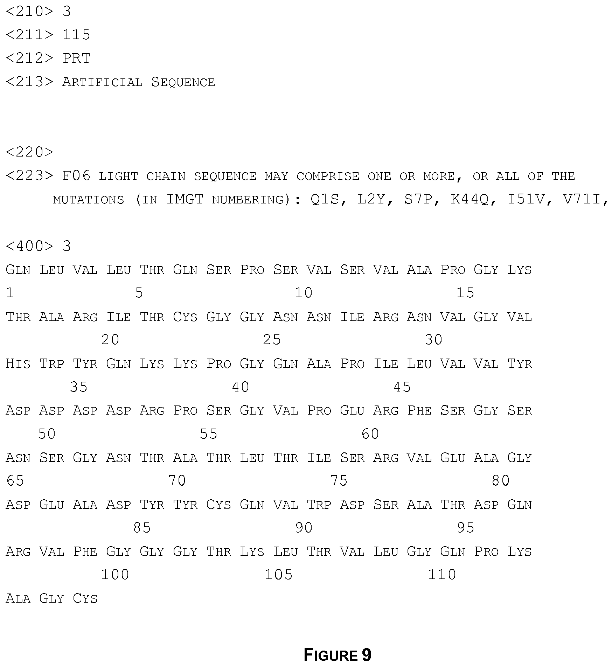

- **Figure 9:**: SEQ ID NO: 3

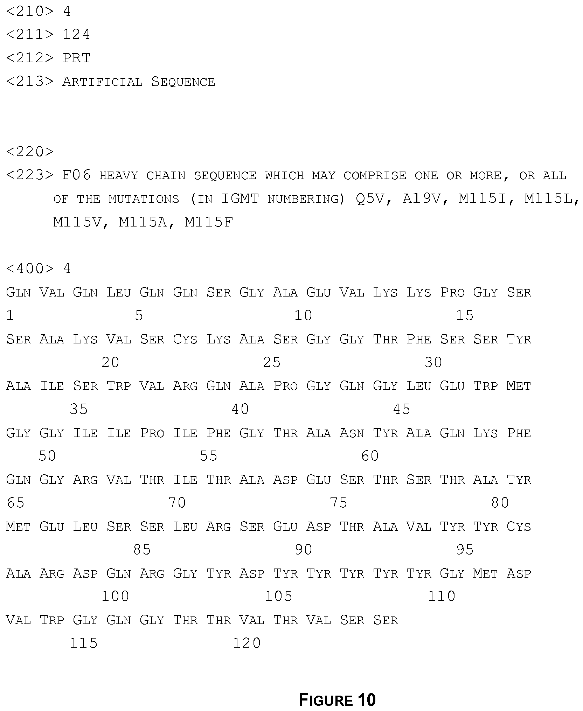

- **Figure 10:**: SEQ ID NO: 4

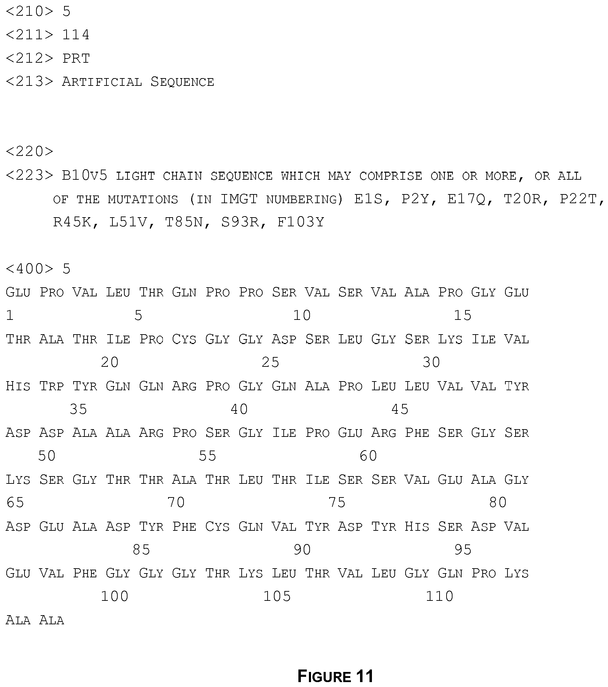

- **Figure 11:**: SEQ ID NO: 5

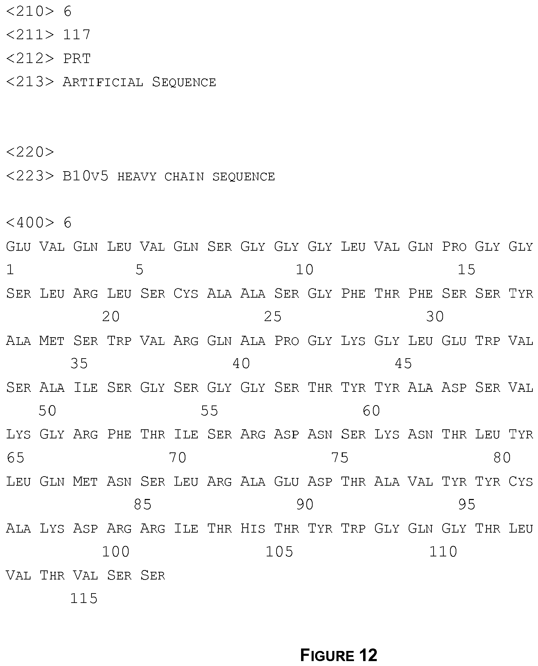

- **Figure 12:**: SEQ ID NO: 6

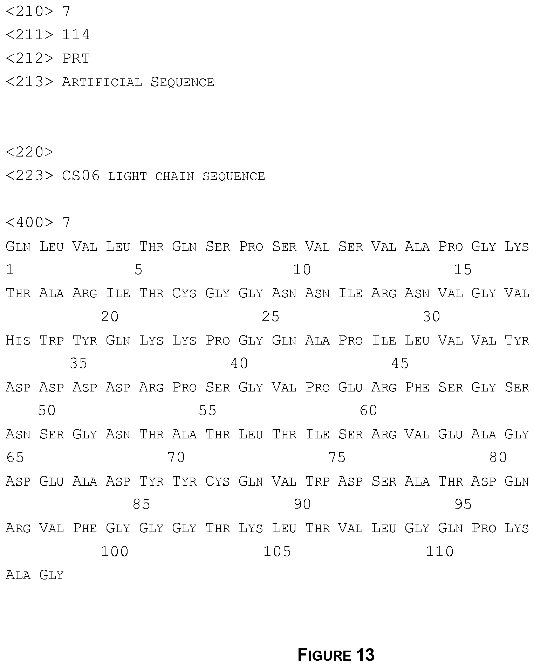

- **Figure 13:**: SEQ ID NO: 7

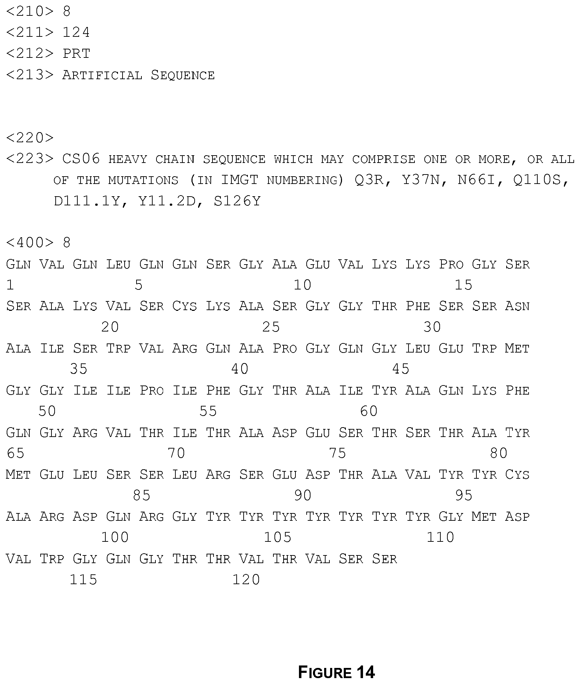

- **Figure 14:**: SEQ ID NO: 8

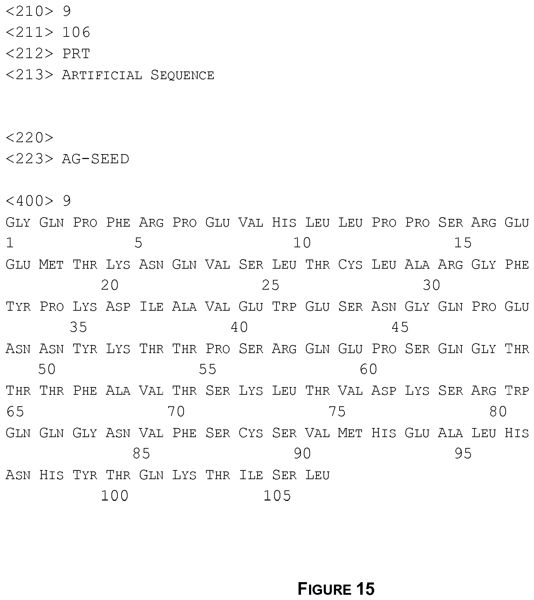

- **Figure 15:**: SEQ ID NO: 9

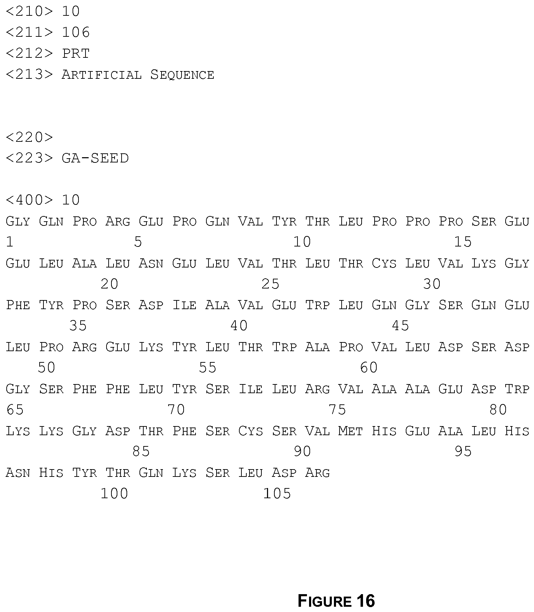

- **Figure 16:**: SEQ ID NO: 10

### SEQUENCE LISTING

| | |
|---|---|
| SEQ ID NO: 1 | anti-c-MET clone B10 light chain amino acid sequence |
| SEQ ID NO: 2 | anti-c-MET clone B10 heavy chain amino acid sequence |
| SEQ ID NO: 3 | anti-c-MET clone F06 light chain amino acid sequence |
| SEQ ID NO: 4 | anti-c-MET clone F06 heavy chain amino acid sequence |
| SEQ ID NO: 5 | anti-c-MET clone B10v5 light chain amino acid sequence |
| SEQ ID NO: 6 | anti-c-MET clone B10v5 heavy chain amino acid sequence |
| SEQ ID NO: 7 | anti-c-MET clone CS06 light chain amino acid sequence |
| SEQ ID NO: 8 | anti-c-MET clone CS06 heavy chain amino acid sequence |
| SEQ ID NO: 9 | AG-SEED |
| SEQ ID NO: 10 | GA-SEED |
| SEQ ID NO: 11 | human MET (c-MET) |

### DETAILED DESCRIPTION OF THE INVENTION

Although the present invention is described in detail below, it is to be understood that this invention is not limited to the particular methodologies, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

In the following, the elements of the present invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine the explicitly described embodiments with any number of the disclosed and/or preferred elements. Furthermore, any permutations and combinations of all described elements in this application should be considered disclosed by the description of the present application unless the context indicates otherwise.

Throughout this specification and the claims which follow, unless the context requires otherwise, the term "comprise", and variations such as "comprises" and "comprising", will be understood to imply the inclusion of a stated member, integer or step but not the exclusion of any other non-stated member, integer or step. The term "consist of" is a particular embodiment of the term "comprise", wherein any other non-stated member, integer or step is excluded. In the context of the present invention, the term "comprise" encompasses the term "consist of".

The terms "a" and "an" and "the" and similar reference used in the context of describing the invention (especially in the context of the claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. No language in the specification should be construed as indicating any non-claimed element essential to the practice of the invention.

Several documents are cited throughout the text of this specification. Each of the documents cited herein (including all patents, patent applications, scientific publications, manufacturer's specifications, instructions, etc.), whether supra or infra, are hereby incorporated by reference in their entirety. Nothing herein is to be construed as an admission that the invention is not entitled to antedate such disclosure by virtue of prior invention.

The described objective is solved by the present invention, namely by the subject matter of the appended claims.

The inventors have surprisingly found that the inventive anti-c-Met antibody or antigen-binding fragment thereof binds c-MET with high affinity can be used to efficiently inhibit c-MET expressing tumors.

The described objectives are solved according to a first embodiment by the inventive anti-c-MET antibodies or antigen-binding fragments thereof, which bind to human c-MET with an affinity of at least 10⁻⁸ M. For example, the inventive c-MET antibodies or antigen binding fragements thereof bind to c-MET with an affinity of at least 10⁻⁸M, e.g. with at least 1x10⁻⁹ M, 2x10⁻⁹ M, 3x10⁻⁹, 4x-10⁻⁹ M, 5x10⁻⁹ M, 6x10⁻⁹M, 7x10⁻⁹M, 8x10⁻⁹M, 9x10⁻⁹M, or with an affinity of at least about 10⁻⁸M to about 10⁻¹⁰M. As used for the inventive anti c-MET antibody the term "antibody" refers to immunoglobulin molecules and immunologically active portions of immunoglobulin molecules, i.e., molecules that contain an antigen binding site that immunospecifically bind an antigen. The term also refers to antibodies comprised of two immunoglobulin heavy chains and two immunoglobulin light chains, or for example, to a monoclonal antibody, a chimeric antibody, a CDR-grafted antibody, a humanized antibody. The term "antigen-binding fragment" as used in the present invention refers to a Fab, a Fab', a F(ab')₂, a Fv, a disulfide linked Fv, a scFv, a single domain antibody (dAb), a diabody, a multispecific antibody, a dual specific antibody, a bispecific antibody, a functionally active epitope-binding fragment thereof and single chains (e.g., Huston et al., Proc. Natl. Acad. Sci. U.S.A., 85, 5879-5883 (1988) and Bird et al., Science 242, 423-426 (1988), which are incorporated herein by reference). (See, generally, Hood et al., Immunology, Benjamin, N.Y., 2ND ed. (1984), Harlow and Lane, Antibodies. A Laboratory Manual, Cold Spring Harbor Laboratory (1988) and Hunkapiller and Hood, Nature, 323, 15-16 (1986), which are incorporated herein by reference). c-MET as used herein refers to MET Proto-Oncogene, Receptor Tyrosine Kinase (UniProtKB database antry P08581), which may also be referred to as Hepatocyte Growth Factor Receptor.

The term Fab fragment refers to an antigen-binding antibody fragment of the inventive antibody which can e.g. be obtained by papain treatment of IgG type immunoglobulins, which will result in two Fab fragment and an Fc domain. Functional aspects and pmthods to obtain Fab fragments are described e.g. in "Applications and Engineering of Monoclonal Antibodies" by D.J. King, CRC Press, 1998, chapter 2.4.1; Zaho et al. Protein Expression and Purification 67 (2009) 182-189; S.M. Andrew, J.A. Titus, Fragmentation of immunoglobulin G, Curr. Protoc. Cell Biol. (2003) Unit 16.14 (Chapter 16). The inventive heterodimeric bispecific immunoglobulin molecule may e.g. also comprise a first scFv fragment that specifically binds to EGFR. The term "scFv" as used in the present invention refers to a molecule comprising an antibody heavy chain variable domain (or region; VH) and an antibody light chain variable domain (or region; VL) connected by a linker, and lacks constant domains, e.g. an scFv fragment according to the invention may e.g. include binding molecules which consist of one light chain variable domain (VL) or portion thereof, and one heavy chain variable domain (VH) or portion thereof, wherein each variable domain (or portion thereof) is derived from the same or different antibodies. scFv molecules preferably comprise an linker interposed between the VH domain and the VL domain, which may e.g. include a peptide sequence comprised of the amino acids glycine and serine. For example, the peptide sequence may comprise the amino acid sequence (Gly₄ Ser)ₙ, whereby n is an integer from 1-6, e.g. n may be 1, 2, 3, 4, 5, or 6, preferably n=4. scFv molecules and methods of obtaining them are known in the art and are described, e.g., in U.S. Pat. No. 5,892,019, Ho et al. 1989. Gene 77:51; Bird et al. 1988 Science 242:423; Pantoliano et al. 1991. Biochemistry 30:10117; Milenic et al. 1991. Cancer Research 51:6363; Takkinen et al. 1991. Protein Engineering 4:837.

According to one embodiment the inventive antibody or antigen-binding fragment thereof as disclosed above binds to e.g. murine or human human c-MET, preferably to human c-MET having the amino acid sequence according to SEQ ID NO: 11, or human c-MET, which includes both full-length c-MET and c-MET with its signal peptide removed, e.g. with amino acids 1-24 cleaved off, including c-MET variant N375S. For example, the inventive antibody or antigen-binding fragment as disclosed above specifically binds to c-MET variant N375S (*rs33917957*)*,* but may e.g. also bind to c-MET variants such as those disclosed in Nat Genet. 1997 May;16(1):68-73, e.g. c-MET R970C (MET^{R970C}), c-MET T992I (MET^{T992I}), MET^{M1149T}, MET^{V1206L}, MET^{V1238I}, MET^{D1246N}, MET^{Y1248C}, MET^{L1213V}, MET^{D1246H}, MET^{Y1248H}, MET^{M1268T}, MET^{A320V}, MET^{N375S} with the signal peptide cleaved off (e.g. amino acids 1-24 of UniProtKB P08581).

In one embodiment the inventive antibody as disclosed above, or the inventive antigen-binding fragment thereof binds to an epitope comprised in the SEMA domain of human c-MET and inhibits c-MET signaling. For example, the inventive antibody, or antigen-binding fragment thereof may bind to an epitope comprised in amino acids 52-496 of mature human c-MET (UniProtKB P08581), e.g to a linear or conformational epitope comprised in amino acids 52 - 496, or e.g. in amino acids 27-515 of human c-MET thereby inhibiting c-MET signaling. The term "epitope" as used in the present invention encompasses both a linear epitope for which in case of a linear epitope the consecutive amino acids are recognized by the antibody as well as a conformational epitope for which the antibodies recognize amino acids to the extent they adopt a proper configuration or conformation within the mature and correctly folded protein. Conformational epitopes are determined by both, the three dimensional structure of a protein, such as e.g. human c-MET, and its primary amino acid sequence. An epitope typically includes at least 3, and more usually, at least 5 or 8, 10 amino acids, e.g. an epitope may comprise 3, 4, 5, 6, 7, 8, 9, or 10 amino acids. Typically, an epitope also is less than 20 residues (e.g., amino acids or nucleotides) in length, such as less than 15 residues or less than 12 residues.

The inventive antibody may e.g. bind to an epitope comprised in the SEMA domain with an affinity of at least 10⁻⁸M, e.g. with at least 1x10⁻⁹ M, 2x10⁻⁹ M, 3x10⁻⁹, 4x-10⁻⁹ M, 5x10⁻⁹ M, 6x10⁻⁹M, 7x10⁻⁹M, 8x10⁻⁹M, 9x10⁻⁹M.

According to one embodiment the inventive antibody or antigen-binding fragment thereof as disclosed above binds to an epitope comprised in immunoglobulin-plexin-transcription (IPT) domains 1-4 of human c-MET and inhibits c-MET signaling. For example, the IPT domains of human c-Met (UniProtKB P08581) comprise amino acids 562-655 (IPT domain 1), 656-739 (IPT domain 2), 741-842 (IPT domain 3) and amino acids 856-952 (IPT domain 4). The inventive antibody may e.g. bind to an epitope comprised in domain 1, IPT domain 2, IPT domain 3 or IPT domain 4 with an affinity as disclosed above, e.g. with an affinity of at least 10⁻⁸M, e.g. with at least 1x10⁻⁹ M, 2x10⁻⁹ M, 3x10⁻⁹, 4x-10⁻⁹ M, 5x10⁻⁹ M, 6x10⁻⁹M, 7x10⁻⁹M, 8x10⁻⁹M, 9x10⁻⁹M. In one aspect, the inventive antibody or antigen-binding fragment thereof binds to IPT domain 1 and inhibits c-Met signaling. The inventive antibody or antigen-binding fragment thereof as disclosed above may e.g. also bind to more than one IPT domain if the epitope is a conformational epitope. For example, the inventive antibody or anti-binding fragment thereof may bind to an epitope which is comprised in IPT domains 1 and 2, or 2 and 3, or 3 and 4, or 1 and 4, or 1 and 3, or 2 and 4, or e.g. in IPT domains 1, 2, 3 and 4.

According to one embodiment inventive antibody or antigen-binding fragment thereof as disclosed above inhibits the binding of recombinant human HGF recombinant to human c-MET extracellular domain (ECD) at a concemtration of 0.9x10⁻⁹M or less, e.g. of about 0.1x10⁻⁹M or less, 0.2x10⁻⁹M or less, 0.3x10⁻⁹M or less, 0.5x10⁻⁹M or less, 0.75x10⁻⁹M or less, by 50% in an enzyme-linked immunosorbent assay (ELISA) using HGF in solid phase. For example, inhibition of HGF binding by the inventive antibody or antigen-binding fragment as disclosed above to human c-MET ECD may be asses using biolayer interferometry the principles of which are described in Analytical Biochemistry 361 (2007) 1-6. For example, different commercially available technologies may be used, such as , Biacore, 2000, 3000, T100, Flexchip, S51, and A100 or dotLab (Axela Biosensors), MultiSPRinter (Toyobo), Proteomic Processor (Lumera), SPRi-Plex (GenOptics), BIND (SRU Biosystems), Epic (Corning), ProteOn XPR (Bio-Rad) each of which may be used according to the manufactuerer's instructions. In one example an Octet Red96 platform (ForteBio) may be used equipped with Octet Data Acquisition and Analysis software. Mesurements may e.g. be carried out at 30°C using a 1000 rpm orbital sensor agitation in a volume of 200 µl in black 96-well microplates following the manufacturer's instructions. For example, ELISA MaxiSorp® plates may be coated with about 1 to about 1.5 pmol recombinant HGF overnight at 4°C followed by a blocking of the Maxisorb® plates with 1-5% BSA (e.g. 1%, 2%, 3%, 4%, 5% BSA) BSA in PBS-T. Biotinylated c-MET ECD at a concentration of about 0.1pmol to about 2pmol, e.g. from about 0.125pmol, 0.15pmol, 0.175pmol, 0.2pmol, 0.25pmol, 0.3pmol, 0.4pmol, 0.5pmol, 0.6pmol, 0.7pmol, 0.8pmol, 0.9pmol, 1.0pmol to about 1.25 pmol, 1.5pmol, 1.75pmol, 2pmol, may then e.g. be incubated with serial dilutions of the inventive antibodies or antigen-binding fragments thereof at a concentration from about 0.2nM to about 200nM, e.g. from about 0.2nM, 0.3nM, 0.4nM, 0.5nM, 0.6nM, 0.7nM, 0.8nM, 0.9nM, 1.0nM, 1,25nM, 1.5nM, 2nM, 2.5nM, 3nM, 3.5nM, 4nM, 5nM, 6nM, 7nM, 8nM, 9nM, 10nM to about 15mM, 17.5nM, 20nM, 25 nM, 30 nM, 32.5nM, 35 nM, 40nM, 42.5nM, 45nM, 47.5nM, 50nM, 55nM, 60nM, 65nM, 70nM, 75nM, 80nM, 85nM, 90nM, 95nM, 100nM, 125nM 150nM, 175nM, 200nM, or e.g. from about 15nM, 17.5nM, 20nM, 25 nM, 30 nM, 32.5nM, 35 nM, 40nM, 42.5nM, 45nM, 47.5nM, 50nM to about 55nM, 60nM, 65nM, 70nM, 75nM, 80nM, 85nM, 90nM, 95nM, 100nM, 125nM 150nM, 175nM, 200nM, or e.g. from about 55nM, 60nM, 65nM, 70nM, 75nM, 80nM, 85nM, 90nM, 95nM, 100nM to about 125nM 150nM, 175nM, 200nM. Binding of biotinylated c-MET ECD to immobilized HGF may then e.g. be visualized using HRP-conjugated streptavidin in a diulution of about 1:100 to about 1:10,000, e.g. 1:250, 1:500, 1:1000, 1:1500, 1:2000, 1:2500, 1:3000, 1:4000, 1:4500, 1:5000, 1: 7,500, 1:8000, 1:9000, followed by e.g. the addition of Ultra TMB ELISA substrate solution and sulfuric acid. The resulting absorbance of c-MET ECD binding to HGF without addition of the inventive anti-c-MET antibodies or antigen-binding fragments thereof may be defined as 100% HGF binding. For example, controls may include anti-Hen Egg Lysozyme (HEL) SEED isotype control antibodies at a concentration of about 0.2nM to about 200nM, e.g. 0.25nM, 5nM, 10nM, 50nM, 75nM, 100nM, 125nM, 150nM, 175 nM. Data may be plotted as % HGF binding against the logarithm of concentration of the inventive antibodies or antigen-binding fragments thereof. For example, the recombinant HGF used in the above assays may be obtained from commercial sources, or e.g. may be manufactured in insect cells as described in Biotechnol. Prog. 2000, 16, 146-151. The c-MET ECD fragment which may e.g. be used in the ELISA assay as disclosed above comprises amino acids 24-963 of human c-MET, or e.g. amino acids 52-952 of human c-MET (e.g. c-MET lacking the signal peptide and the transmembrane domain, or e.g. a commercially available c-MET ECD-Fc protein).

In one embodiment antigen-binding fragment of the inventive antibody as disclosed above are e.g. a Fab fragment, or a F(ab')₂ fragment, or a scFv fragment which have the same binding properties as the inventive antibody as disclosed above. The term "antigen-binding fragment" as used in the present invention may also refers to a Fd fragment consisting of the VH and CH1 domains, or a Fv fragment consisting of the VL and VH domains of a single arm of an antibody, or a dAb fragment (see e.g. Ward et al (1989) Nature 341 544-46), which comprises a VH domain, or e.g. an isolated complementarity determining region (CDR) of the the light and heavy chains comprised in any of SEQ ID NO: 1 - SEQ ID NO: 8 of the inventive antibodies. The term "scFv" as used in the present invention refers to a molecule comprising an antibody heavy chain variable domain (or region; VH) and an antibody light chain variable domain (or region; VL) connected by a linker, and lacks constant domains, e.g. an scFv fragment according to the invention may e.g. include binding molecules which consist of one light chain variable domain (VL) or portion thereof, and one heavy chain variable domain (VH) or portion thereof, wherein each variable domain (or portion thereof) is derived from the same or different antibodies. scFv molecules preferably comprise an linker interposed between the VH domain and the VL domain, which may e.g. include a peptide sequence comprised of the amino acids glycine and serine. For example, the peptide sequence may comprise the amino acid sequence (Gly₄ Ser)ₙ, whereby n is an integer from 1-6, e.g. n may be 1, 2, 3, 4, 5, or 6, preferably n=4. scFv molecules and methods of obtaining them are known in the art and are described, e.g., in U.S. Pat. No. 5,892,019, Ho et al. 1989. Gene 77:51; Bird et al. 1988 Science 242:423; Pantoliano et al. 1991. Biochemistry 30:10117; Milenic et al. 1991. Cancer Research 51:6363; Takkinen et al. 1991. Protein Engineering 4:837. The term "di-scFv" as used for the inventive antigen-binding fragments refers to two scFv fragments which are coupled to each other via a linker, e.g. such as disclosed in Cancer Research 54, 6176-618,. December 1, 1994, or Chem Commun (Camb). 2007 Feb 21;(7):695-7. Antigen-binding fragments of the inventive antibodies may e.g. also include diabodies, whereby the term "diabodies" refers to a small antibody fragments with two antigen-binding sites, which fragments comprise a heavy chain variable domain (VH) connected to a light chain variable domain (VL) in the same polypeptide chain (VH-VL). By using a linker that is too short to allow pairing between the two domains on the same chain, the domains are forced to pair with the complementary domains of another chain and create two antigen-binding sites (see e.g. EP 0 404097 B1; WO 93/11161, and Hollinger et al., Proc. Natl. Acad. Sci. USA 90: 6444-6448 (1993)). For example, antigen-binding fragments according to the invention may be obtained by digestion with peptidases such as pepsin, or papain: Pepsin will result in proteolytic cleavage below the disulfide linkages and result in a F(ab')₂ antibody fragments, while proteolytic cleavage by papain, which cleaves above the disulfide linkages, will result in two Fab fragments. Accordingly, a F(ab')₂ fragment is a dimer of Fab which itself is a light chain joined to V_{H}-C_{H1} by a disulfide bond. The F(ab')₂ may be reduced under mild conditions to break the disulfide linkage in the hinge region, thereby converting the F(ab)'₂ dimer into a Fab' monomer. The aforementioned antibody fragments are defined in terms of the digestion of an intact antibody with pepsin and papain, however, such fragments may be synthesized *de novo* either chemically or by using recombinant DNA methodology.

The inventive antibody is an IgG type antibody. The term "IgG type antibody" as used for the inventive antibody refers to IgG class antibodies, which in humans includes four subclasses (IgG1, IgG2, IgG3, and IgG4). (Alberts, B. et al., Chapter 23: The Immune System, In Molecular Biology of the Cell, 3d Edition, Garland Publishing, Inc., New York, N.Y.). The inventive antibody may thus be an IgG1, IgG2, IgG3, or IgG4 type antibody, preferably the inventive antibody is an IgG1 type antibody. The inventive IgG1 type antibody may for example further comprise mutations in its Fc region such as those disclosed in Duncan et al., Nature 332:563 (1988), Sondermann et al., Nature 406:267 (2000); Wines et al., J. Immunol. 164:5313 (2000); Canfield and Morrison, J. Exp. Med. 173:1483 (1991); Tao et al., J. Exp. Med. 178:661 (1993), e.g. amino acids at EU index positions 330 and 331, or e.g. substitutions at EU index positions 234, 235, and 237 to reduce effector functions mediated by the Fc by reducing FcyRI, FcgRlla, or Fcglll binding and/or complement C1q binding.

According to one embodiment the inventive antibody or antigen-binding fragment thereof comprises at least one of the amino acid sequences according to SEQ ID NO: 1, SEQ ID NO: 2, SEQ ID NO: 3, SEQ ID NO: 4, SEQ ID NO: 5, SEQ ID NO: 6, SEQ ID NO: 7, or SEQ ID NO: 8. For example, the light chains of the inventive antibody or antigen-binding fragment thereof may comprise at least one of the amino acid sequence according to ID NO: 1, SEQ ID NO: 3, SEQ ID NO: 5, or SEQ ID NO: 7 and the heavy chain may comprise at least one of the amino acid sequence according to SEQ ID NO: 2, SEQ ID NO: 4, or SEQ ID NO: 6, or SEQ ID NO: 8.

The inventive antibody comprises light and heavy chains comprising the amino acid sequences according to SEQ ID NO: 1 and SEQ ID NO: 2, or SEQ ID NO: 3 and SEQ ID NO: 4, or SEQ ID NO: 5 and SEQ ID NO: 6, or SEQ ID NO: 7 and SEQ ID NO:8. The light and heavy chains of the inventive antibody as disclosed above, may e.g. also comprise kinetic variants comprising one or more, e.g. one, two, three, or more of the following mutations in their respective amino acid sequence according to the EU index (EU numbering): For example, the inventive light and heavy chains of the antibodies or antigen-binding fragments thereof as disclosed above may comprise kinetic mutations, which may e.g. alter the dissociation rate of the inventive antibody upon binding to c-MET. For example, the light chain sequence according to SEQ ID NO: 1 may comprise one or more, e-g- one, two, three, four, five, six, seven, eight, nine, ten, eleven, or all of the following mutations: V3A, T11V, T14S, R18S, R43Q, L45P, E74D, T85N, S86T, A90T, T92S, G100A, whereby the numbering is provided according to IMGT numbering. The heavy chain sequence according to SEQ ID NO: 2 may comprise the mutation Q6E according to IMGT numbering, e.g. the light chain sequence according to SEQ ID NO: 3 may comprise one or more, e.g. one, two, three, four, or all of the mutations (in IMGT numbering): Q1S, L2Y, S7P, K44Q, I51V, V71I, or e.g. the heavy chain sequence according to SEQ ID NO: 4 may comprise one or more, e.g. one, two, three, four, five, six, or all of the mutations (in IGMT numbering) Q5V, A19V, M115I, M115L, M115V, M115A, M115F, or e.g. the light chain sequence according to SEQ ID NO: 5 may comprise one or more, e.g. one, two, three, four, five, six, seven, eight, or all of the mutations (in IMGT numbering) E1S, P2Y, E17Q, T20R, P22T, R45K, L51V, T85N, S93R, F103Y, or e.g. the heavy chain sequence according to SEQ ID NO: 8 may comprise one or more, e.g. one, two, three, four, five, six, or all of the mutations (in IMGT numbering) Q3R, Y37N, N66I, Q110S, D111.1Y, Y11.2D, S126Y. The light and heavy chain sequences as disclosed above may e.g. both comprise one or more of the mutations disclosed above, or e.g. only the heavy, or the light chain sequences as disclosed above comprised in the inventive antibody or antigen-binding fragment thereof may comprise one or more, or all of the mutations disclosed above. Accordingly, the inventive antibody or antigen-binding fragment thereof as disclosed above may comprise light and heavy chain sequences which comprise one or more (e.g. one, two, three, or four) of the mutations disclosed above.

In one example the inventive antibody is an IgG type antibody and comprises the light and heavy chains that comprise amino acid sequences according to SEQ ID NO: 7 and SEQ ID NO: 8 (CS06), or SEQ ID NO: 5 and SEQ ID NO: 6 (B10v5) as disclosed above and binds to human c-MET with an affinity of at least 10⁻⁹M, e.g. 3x10⁻¹⁰, 4x-10⁻¹⁰ M, 5x10⁻¹⁰ M, 6x10⁻¹⁰M, 7x10⁻¹⁰M, 8x10⁻¹⁰M, 9x10⁻¹⁰M. In one example, the inventive heterodimeric immunoglobulin molecule is a biparatopic bispecific heterodimeric immunoglobulin molecule, which comprises a first Fab or scFv fragment comprising SEQ ID NO: 7 and SEQ ID NO: 8 and a second Fab or scFv fragment comprising SEQ ID NO: 5 and SEQ ID NO: 6, whereby the first Fab or scFv fragment may be fused to an AG-SEED and the second Fab or scFv fragment may be fused to a GA-SEED, or e.g. whereby the first Fab or scFv fragment may be fused to a GA-SEED and the second Fab or scFv may be fused to an AG-SEED. The term "SEED" as used for, or in the context of the inventive heterodimeric molecule refers to strand-exchange engineered domain (SEED) C_{H}3 heterodimers as disclosed in WO2007/110205 A2, Protein Engineering, Design & Selection vol. 23 no. 4 pp. 195-202, 2010. These heterodimeric molecules are derivatives of human IgG and IgA CH3 domains and create complementary human SEED C_{H}3 heterodimers that are composed of alternating segments of human IgA and IgG C_{H}3 sequences. The resulting pair of SEED C_{H}3 domains preferentially associates to form heterodimers in a 1:1 ratio when expressed in mammalian cells to form "SEEDbodies" (Sb). The term "GA-SEED" hereby indicates that the SEED molecule begins with an IgG sequence, followed by an IgA sequence, while "AG-SEED" refers to the fact that the SEED molecule begins with an IgA-derived sequence followed by an IgG-derived sequence. For example, the a biparatopic bispecific heterodimeric immunoglobulin molecule bind to human c-MET with an affinity of at least 1x10⁻⁹M, e.g. with at least 2x10⁻¹¹, 3x-10⁻¹¹ M, 4x10⁻¹¹ M, 5x10⁻¹¹M, 6x10⁻¹¹M, 7x10⁻¹¹M, 8x10⁻¹¹M, 9x10⁻¹¹M).

According to one embodiment, the inventive antibody or antigen-binding fragment thereof as disclosed above is further coupled to a diagnostic or therapeutic agent. The term diagnostic agent as used for the inventive antibody or antigen-binding fragment thereof refers to an entity which can be used to detect the inventive antibody or antigen-binding fragment thereof specifically bound to c-MET, preferably human c-MET and/or c-MET variants as disclosed above. For example, the diagnostic agent may be a radioactive isotope, fluorecent probes, fluorophore, chemiluminesceres, enzymes, enzyme substrates, enzyme cofactors, enzyme inhibitors, dyes, metal ions, or biotin, or streptavidin, which allow detection of the inventive antibody or antigen-binding fragment thereof bound to c-MET. The term "coupled" as used for the inventive antibody or antigen-binding fragment thereof refers to the fact that the dye, radioisotope, fluorecent probes, fluorophore, chemiluminesceres, enzymes, enzyme substrates, enzyme cofactors, enzyme inhibitors, dyes, metal ions, biotin, or streptavidin may e.g. be non-covalently attached or boun via ionic, or hydrophobic interactions, or covalently attached to inventive antibody or antigen-binding fragment thereof. For example, coupling of the detecable labels as disclosed above such as fluorecent probes, dyes, or enzymes to the inventive antibody or antigen-binding fragment thereof as disclosed above may be done according to methods known in the art such as those disclosed in Methods Cell Biol. 2001;63:185-204; Methods Mol Biol. 2010;588:43-8; Curr Protoc Mol Biol. 2001 May; Chapter 11:Unit 11.1.

Examples for a detectable label according to the invention which may coupled to the inventive antibody or antigen-binding fragment thereof include alkaline phosphatase, horseradish peroxidase, beta-galactosidase, Tobacco Etch Virus nuclear-inclusion-a endopeptidase ("TEV protease"). Fluorophores which may e.g. be coupled to the inventive antibody or antigen-binding fragment thereof as disclosed above may be one of 1,8-ANS, 4-methylumbelliferone, 7-amino-4-methylcoumarin, 7-hydroxy-4-methylcoumarin, Acridine, Alexa Fluor 350™, Alexa Fluor 405™, AMCA, AMCA-X, ATTO Rho6G, ATTO Rho11, ATTO Rho12, ATTO Rho13, ATTO Rho14, ATTO Rho101, Pacific Blue, Alexa Fluor 430™, Alexa Fluor 480™, Alexa Fluor 488™, BODIPY 492/515, Alexa Fluor 532™, Alexa Fluor 546™, Alexa Fluor 555™, Alexa Fluor 594™, BODIPY 505/515, Cy2, cyQUANT GR, FITC, Fluo-3, Fluo-4, GFP (EGFP), mHoneydew, Oregon Green™ 488, Oregon Green™ 514, EYFP, DsRed, DsRed2, dTomato, Cy3.5, Phycoerythrin (PE), Rhodamine Red, mTangerine, mStrawberry, mOrange, mBanana, Tetramethylrhodamine (TRITC), R-Phycoerythrin, ROX, DyLight 594, Calcium Crimson, Alexa Fluor 594™, Alexa Fluor 610™, Texas Red, mCherry, mKate, Alexa Fluor 660™, Alexa Fluor 680™ allophycocyanin, DRAQ-5, carboxynaphthofluorescein, C7, DyLight 750, Cellvue NIR780, DM-NERF, Eosin, Erythrosin, Fluorescein, FAM, Hydroxycoumarin, IRDyes (IRD40, IRD 700, IRD 800), JOE, Lissamine rhodamine B, Marina Blue, Methoxy coumarin, Naphtho fluorescein, PyMPO, 5-carboxy-4',5'-dichloro-2',7'-dimethoxy fluorescein, 5- carboxy-2',4',5',7'-tetrachlorofluorescein, 5-carboxyfluorescein, 5-carboxyrhodamine, 6- carboxyrhodamine, 6-carboxytetramethyl amino, Cascade Blue, Cy2, Cy3, Cy5,6-FAM, dansyl chloride, HEX, 6-JOE, NBD (7-nitrobenz-2-oxa-1,3-diazole), Oregon Green 488, Oregon Green 500, Oregon Green 514, Pacific Blue, phthalic acid, terephthalic acid, isophthalic acid, cresyl fast violet, cresyl blue violet, brilliant cresyl blue, para- aminobenzoic acid, erythrosine, phthalocyanines, azomethines, cyanines, xanthines, succinylfluoresceins, rare earth metal cryptates, europium trisbipyridine diamine, a europium cryptate or chelate, diamine, dicyanins, or La Jolla blue dye. Flurophores which may e.g. be coupled to the inventive antibody or antigen-binding fragment as disclosed above may also include quantum dots. The term quantum dot as used in the present invention refers to a single spherical nanocrystal of semiconductor material where the radius of the nanocrystal is less than or equal to the size of the exciton Bohr radius for that semiconductor material (the value for the exciton Bohr radius can be calculated from data found in handbooks containing information on semiconductor properties, such as the CRC Handbook of Chemistry and Physics, 83rd ed., Lide, David R. (Editor), CRC Press, Boca Raton, Fla. (2002)). Quantum dots are known in the art, as they are described in references, such as Weller, Angew. Chem. Int. Ed. Engl. 32: 41-53 (1993), Alivisatos, J. Phys. Chem. 100: 13226-13239 (1996), and Alivisatos, Science 271: 933-937 (1996). Quantum dots may e.g. be from about 1 nm to about 1000 nm diameter, e.g. 10nm, 20 nm, 30 nm, 40 nm, 50 nm, 60 nm, 70 nm, 80 nm, 90 nm, 100 nm, 150 nm, 200 nm, 250 nm, 300 nm, 350 nm, 400 nm, 450 nm, or 500 nm, preferably at least about 2 nm to about 50 nm, more preferably QDs are at least about 2 nm to about 20 nm in diameter (for example about 2, 3, 4, 5, 6, 7, 8, 9, 10, 11, 12, 13, 14, 15, 16, 17, 18, 19, or 20 nm). QDs are characterized by their substantially uniform nanometer size, frequently exhibiting approximately a 10% to 15% polydispersion or range in size. A QD is capable of emitting electromagnetic radiation upon excitation (i.e., the QD is photoluminescent) and includes a "core" of one or more first semiconductor materials, and may be surrounded by a "shell" of a second semiconductor material. A QD core surrounded by a semiconductor shell is referred to as a "core/shell" QD. The surrounding "shell" material will preferably have a bandgap energy that is larger than the bandgap energy of the core material and may be chosen to have an atomic spacing close to that of the "core" substrate. The core and/or the shell can be a semiconductor material including, but not limited to, those of the groups II-VI (ZnS, ZnSe, ZnTe, US, CdSe, CdTe, HgS, HgSe, HgTe, MgS, MgSe, MgTe, CaS, CaSe, CaTe, SrS, SrSe, SrTe, BaS, BaSe, BaTe, and the like) and III-V (GaN, GaP, GaAs, GaSb, InN, InP, InAs, InSb, and the like) and IV (Ge, Si, and the like) materials, PbS, PbSe, and an alloy or a mixture thereof. Preferred shell materials include ZnS. Quantum dots may e.g. be coupled to the inventive antibody or antigen-binding fragment thereof by any method known in the art such as the methods disclosed in Nanotechnology. 2011 Dec 9;22(49):494006; Colloids and Surfaces B: Biointerfaces 84 (2011) 360-368.

In one embodiment, the inventive antibody or antigen-binding fragment thereof may e.g. be coupled to a radioisotope such as ⁴⁷Ca, ¹⁴C, ¹³⁷Cs, ¹⁵⁷Cr, ⁵⁷Co, ⁶⁰Co, ⁶⁷Cu, ⁶⁷Ga, ¹²³I, ¹²⁵I, ¹²⁹I, ¹³¹I, ³²P, ⁷⁵Se, ⁸⁵Sr, ³⁵S, ²⁰¹Th, or ³H, preferably, the radioisotopes are incorporated into a further moelcule, such as e.g. a chelator. Typical chelators that may e.g. be used as a further molecule covalently bound to the amino donor-comprising substrate of the invention are DPTA, EDTA (Ethylenediamine-tetraacetic acid), EGTA (Ethyleneglycol-O, O'-bis(2-aminoethyl)-N, N, N', N'-tetraacetic acid, NTA (Nitrilotriacetic acid), HEDTA (N-(2- Hydroxyethyl)-ethylenediamine-N,N',N'-triacetic acid), DTPA (2-[Bis[2-[bis(carboxymethyl)amino]-ethyl]amino]acetic acid), or DOTA (1,4,7,10-tetraazacyclo-dodecane-1,4,7,10-tetraacetic acid).

In one embodiment the inventive antibody or antigen-binding fragment thereof may be coupled to a therapeutic agent. The term "therapeutic agent" according to the invention, e.g. as used for the inventive antibody or antigen-binding fragment thereof, refers to any compound useful for therapeutic purposes. For example, a therapeutic agent or compound of the invention may be any compound that is administered to a patient for the treatment of a malignancy, such as e.g. cancer. Examples of cancer include, but are not limited to, non-small-cell lung cancer (NSCLC), mesothelioma, unresectable mesothelioma, breast cancer, adrenocarcinoma of stomach or GEJ, gastric, Thymoma, ovarian cancer, adenoid cystic carcinoma, metastatic adenoid cystic carcinoma, bladder cancer, clear cell kidney cancer, head/neck squamous cell carcinoma, lung squamous cell carcinoma, malignant melanoma, ovarian cancer, pancreatic cancer, prostate cancer, renal cell cancer, small-cell lung cancer (SCLC) or triple negative breast cancer, lymphoproliferative disorders, acute lymphoblastic leukemia (ALL), acute myeloid leukemia (AML), chronic lymphocytic leukemia (CLL), chronic myeloid leukemia (CML), diffuse large B-cell lymphoma (DLBCL), EBV-positive DLBCL, primary mediastinal large B-cell lymphoma, T- cell/histiocyte-rich large B-cell lymphoma, follicular lymphoma, Hodgkin's lymphoma (HL), mantle cell lymphoma (MCL), multiple myeloma (MM), myeloid cell leukemia-1 protein (Mcl-1), myelodysplastic syndrome (MDS), non-Hodgkin's lymphoma (NHL), or small lymphocytic lymphoma (SLL), Merkel cell carcinoma (MCC), or squamous head and neck cancer (SHNC).

Therapeutic agents include but are not limited to hydrophilic and hydrophobic compounds. Accordingly, therapeutic agents may e.g. include drug-like molecules, proteins, peptides, antibodies, antibody fragments, aptamers and small molecules. Protein therapeutic agents include e.g. peptides, enzymes, structural proteins, receptors and other cellular or circulating proteins as well as fragments and derivatives thereof, the aberrant expression of which gives rise to one or more disorders. For example, therapeutic agents according to the invention also include, as one specific embodiment, chemotherapeutic agents, cytostatic, or cytotoxic agents. For example, cytostatic agents that may be coupled to the inventive antibody or antigen-binding fragment thereof are one of alkylating agents, antimetabolites, antibiotics, mitotic inhibitors, hormones, or hormone antagonists. Alkylating agents may e.g. include Busulfan (Myleran), Carboplatin (Paraplatin), Chlorambucil, Cisplatin, Cyclophosphamide (Cytoxan), Dacarbazine (DTIC-Dome), Estramustine Phosphate, Ifosphamide, Mechlorethamine (Nitrogen Mustard), Melphalan (Phenylalanine Mustard), Procarbazine, Thiotepa, Uracil Mustard, antimetabolites may e.g. include Cladribine, Cytarabine (Cytosine Arabinoside), Floxuridine (FUDR, 5-Fluorodeoxyuridine), Fludarabine, 5-Fluorouracil (5FU), Gemcitabine, Hydroxyurea, 6-Mercaptopurine (6MP), Methotrexate (Amethopterin), 6-Thioguanine, Pentostatin, Pibobroman, Tegafur, Trimetrexate, Glucuronate, antibiotics may e.g. include Aclarubicin, Bleomycin, Dactinomycin (Actinomycin D), Daunorubicin, Doxorubicin (Adriamycin), Epirubicin, Idarubicin, Mitomycin C, Mitoxantrone, Plicamycin (Mithramycin), or mitotic inhibitors may e.g. include Etoposide (VP-16, VePesid), Teniposide (VM-26, Vumon), Vinblastine, Vincristine, Vindesine, hormones, or hormone antagonists which may e.g. be used include Buserelin, Conjugate Equine Estrogen (Premarin), Cortisone, Chlorotriansene (Tace), Dexamethasone (Decadron), Diethylstilbestrol (DES), Ethinyl Estradiol (Estinyl), Fluoxymesterone (Halotestin), Flutamide, Goserelin Acetate (Zoladex), Hydroxyprogesterone Caproate (Delalutin), Leuprolide, Medroxyprogesterone Acetate (Provera), Megestrol Acetate (Megace), Prednisone, Tamoxifen (Nolvadex), Testolactone (Teslac), Testosterone. Cytostatic or antineoplastic compounds such as those disclosed above are known in prior art and may e.g. be found in D. S. Fischer & T. M. Knobf (1989), The cancer chemotherapy handbook (3rd ed.). Chicago: Year Book Medical and Association of Community Cancer Centers (Spring, 1992), Compendia-based drug bulletin, Rockville, MD. The diagnostic or therapeutic agent as disclosed above, e.g. the detectable label as disclosed above, or the therapeutic agent as disclosed above, may e.g. be coupled to at least one light chain of the inventive antibody, or to at least one heavy chain of the inventive antibody. For example, the diagnostic or therapeutic agent as disclosed above may be attached to one light chain, or to each of the light chains of the inventive antibody, or e.g. to one heavy chain, or each of the heavy chains of the inventive antibody, antigen-binding fragment thereof.

In one aspect, the diagnostic or therapeutic agent may be covalently attached to the inventive antibody or antigen-binding fragment thereof by e.g. selective chemical modification of cysteine or histidine residues in the inventive antibody or antigen-binding fragment thereof. For example, the hinge-region disulfides of the inventive antibody can be selectively reduced to make free sulfhydryls available for targeted labeling. For example, site-specific Reduction with Mercaptoethylamine (MEA) may be done as described in J. Biolg. Chemistry Vol. 275, No. 39, Issue of September 29, pp. 30445-30450, 2000. For example, MEA (Pierce) may be dissolved in 0.1 M sodium phosphate, pH 6.0, 5 mM DTPA at a concentration of 50 mM. and then added to a solution in a 10-fold excess over the inventive antibody concentration (e.g. 300 µM). The reduction may then be allowed to proceeded at room temperature for e.g. 60 min. Following reduction, the inventive antibody solution may be passed through a Bio-Spin 30 column which may be pre-equilibrated in 0.1 M TMAP, pH 8.2, 25 µM DTPA for 2 min at 150 x g. Coupling of the therapeutic or diagnostic agent as disclosed above may then be done in a reduction reaction using mercaptoethylamin in e.g. 0.1M tetramethylammonium phosphate, pH 8.5 with no diethylenetriaminepentaacetic acid present at room temperature for about 20 minutes. The inventive antibody may e.g. be present in a 10-fold excess to the therapeutic or diagnostic agent as disclosed above, e.g. the inventive antibody may be present at a concentration of about 200µM. Unreacted reagents may e.g. be removed by centrifugation using Bio-Spin 30 columns pre-equilibrated with 0.1 M ammonium acetate, pH 6.5, for 2 minutes at 150xg, whereby the centrifugation may be repeated until all small molecular weight materials are removed. Alternatively, site-specific conjugation as described in Methods Mol Biol. 2013;1045:189-203 using thiol-reactive linkers may be used for coupling the diagnostic or therapeutic agent as disclosed above to the inventive antibodies and/or antigen-binding fragments thereof as disclosed above.. In one aspect, the diagnostic or therapeutic agents as disclosed above may e.g. coupled to the inventive antibody or antigen-binding fragment using using enzyme-mediated bioconjugation. For example, sortase A (srtA), or transglutaminase (TGase)-mediated coupling bioconjugations may be used to coupled the diagnostic or therapeutic agent to the inventive antibody or antigen-binding fragment thereof (see e.g. Biomolecules 2013, 3, 870-888; WO2012059882 A1, WO2014145441 A1). SEEDbodies may e.g. also be modified in an analogous fashion using the techniques disclosed above

According to one embodiment a heterodimeric immunoglobulin molecule comprises a first and/or second Fab or scFv fragment which specifically binds to human c-MET as disclosed above, and an antibody hinge region, an antibody C_{H}2 domain and an antibody C_{H}3 domain comprising a hybrid protein-protein interaction interface domain wherein each of said interaction interface domain is formed by amino acid segments of the CH3 domain of a first member and amino acid segments of the CH3 domain of said second member, wherein said protein-protein interface domain of the first chain is interacting with the protein-protein-interface of the second chain by homodimerization of the corresponding amino acid segments of the same member of the immunoglobulin superfamily within said interaction domains, wherein the first engineered immunoglobulin chain or member comprises the polypeptide sequence ("AG-SEED"):
GQPFRPEVHLLPPSREEMTKNQVSLTCLARGFYPKDIAVEWESNGQPENNYKTTPSRQEP SQGTT TFAVTSKLTVDKSRWQQGNVFSCSVMHEALHNHYTQKTISL and the second engineered immunoglobulin chain or member comprises the polypeptide sequence ("GA-SEED"):
GQPREPQVYTLPPPSEELALNELVTLTCLVKGFYPSDIAVEWLQGSQELPREKYLTWAPVL DSDGSFFLYSILRVAAEDWKKGDTFSCSVMHEALHNHYTQKSLDR and wherein the first and/or second Fab or scFv fragment comprise at least two of the amino acid sequences according to SEQ ID NO: 1, SEQ IDNO: 2, SEQ ID NO: 3, SEQ ID NO: 4, SEQ ID NO: 5, SEQ ID NO: 6, SEQ ID NO: 7, or SEQ ID NO: 8.

For example, the heterodimeric immunoglobulin molecule may comprise one Fab, or scFv fragment which comprises SEQ ID NO: 1 and SEQ ID NO: 2, or e.g. SEQ ID NO: 3 and SEQ ID NO 4, or e.g. SEQ ID NO: 5 and SEQ ID NO: 6, or e.f. SEQ ID NO: 7 and SEQ ID NO: 8 as disclosed above. The heterodimeric immunoglobulin molecule as disclosed above may e.g. comprise two Fab, or two scFv, or one Fab and one scFv, each of which may comprise NO: 1 and SEQ ID NO: 2, or e.g. SEQ ID NO: 3 and SEQ ID NO 4, or e.g. SEQ ID NO: 5 and SEQ ID NO: 6, or e.g. SEQ ID NO: 7 and SEQ ID NO:8. For example, the first Fab may comprise the amino acid sequence according to SEQ ID NO: 1 and SEQ ID NO: 2, and the second Fab may comprise the amino acid sequence according to SEQ ID NO: 3 and SEQ ID NO 4, or e.g. the first Fab may comprise the amino acid sequence according to SEQ ID NO: 5 and SEQ ID NO: 6 and the second Fab may comprise the amino acid sequence according to SEQ ID NO: 1 and SEQ ID NO: 2, or e.g. SEQ ID NO: 3 and SEQ ID NO: 4, e.g. the first Fab may comprise the amino acid sequence according to SEQ ID NO: 7 and SEQ ID NO: 8 and the second Fab may e.g. comprise the amino acid sequence according to SEQ ID NO: 1 and SEQ ID NO: 2, or SEQ ID NO: 3 and SEQ ID NO: 4, or e.g. SEQ ID NO: 5 and SEQ ID NO: 6, or in one example the first Fab may comprise the light and heavy chain sequences according to SEQ ID NO: 1 and SEQ ID NO: 2, or SEQ ID NO: 3 and SEQ ID NO: 4, or e.g. SEQ ID NO: 5 and SEQ ID NO: 6 and the second Fab comprises the heavy and light chain sequences according to SEQ ID NO: 7 and SEQ ID NO: 8. In one example, the heterodimeric immunoglobulin molecule according to the invention as disclosed above comprises only one Fab, or scFv, e.g.may comprise a Fab-GA-SEED and a AG-SEED devoid of a Fab or scFv, or e.g. a scFv-GA-SEED and a AG-SEED devoid of a Fab or scFv, or e.g. a GA-SEED devoid of a Fab or scFv and a Fab-AG-SEED, or e.g. GA-SEED devoid of a Fab or scFv and a scFv-AG-SEED, whereby the Fab, or scFv fragments comprise the following light and heavy chain pairs SEQ ID NO: 1 and SEQ ID NO: 2, or e.g. SEQ ID NO: 3 and SEQ ID NO 4, or e.g. SEQ ID NO: 5 and SEQ ID NO: 6, or e.f. SEQ ID NO: 7 and SEQ ID NO: 8, which may further include the mutations as disclosed above. In one embodiment the inventive antigen-binding fragments thereof as disclosed above may e.g. be further fused to a GA-SEED, or AG-SEED to form a SEEDbody upon heterodimerization which e.g. comprises two inventive antigen-binding fragments as disclosed above. For example, an inventive Fab, or scFv may be covalently fused to a GA-SEED, or alternatively to an AG-SEED via a peptide bond to a peptide linker as disclosed herein, whereby the Fab, or scFv comprises the inventive light chain and heavy chain sequences as disclosed above, e.g. the Fab or scFv may comprise one of the following light chain and heavy chain combinations SEQ ID NO: 1, SEQ ID NO: 2; SEQ ID NO: 3, SEQ ID NO: 4; SEQ ID NO: 5, SEQ ID NO: 6; or SEQ ID NO: 7, SEQ ID NO: 8. An inventive SEEDbody may e.g. comprise a GA-SEED fused to anti-cMET B10 (comprising the amino acid sequence according to SEQ ID NOs: 1, 2) and an AG-SEED fused to anti-cMET clone F06 (comprising the amino acid sequence according to SEQ ID NOs: 3, 4), or e.g. comprise a GA-SEED fused to anti-cMET clone B10v5 (comprising the amino acid sequence according to SEQ ID NOs: 5, 6) and an AG-SEED fused to anti-cMET clone CS06 (comprising the amino acid sequence according to SEQ ID NOs: 7, 8), or e.g.an AG-SEED fused to anti-cMET B10 (comprising the amino acid sequence according to SEQ ID NOs: 1, 2) and a GA-SEED fused to anti-cMET clone F06 (comprising the amino acid sequence according to SEQ ID NOs: 3, 4), or e.g. comprise an AG-SEED fused to anti-cMET clone B10v5 (comprising the amino acid sequence according to SEQ ID NOs: 5, 6) and a GA-SEED fused to anti-cMET clone CS06 (comprising the amino acid sequence according to SEQ ID NOs: 7, 8), whereby the light and heavy chains of the SEEDbodies may be attached to a diagnostic or therapeutic agent as disclosed above. For example, the inventive SEEDbody may comprise one, two, three, or four light chains coupled or attached to a therapeutic or diagnostic agent as disclosed above and/or one, two, three, or four heavy chains coupled or attached to a therapeutic or diagnostic agent as disclosed above. The SEEDbodies as disclosed above may e.g. according to one embodiment encompass kinetic variants as disclosed above comprising one or more, e.g. one, two, three, or more of the mutations disclosed above in their respective amino acid sequence.

In one embodiment, the inventive antibody as disclosed above, or the heterodimeric immunoglobulin molecule as disclosed above are coupled to a cytotoxin. For example, cytotoxins coupled to the inventive antibody or heterodimeric immunoglobulin molecule as disclosed above may e.g. also be referred to as "payloads". Cytotoxins which may e.g. be used according to the invention can be grouped into two main classes: The first class includes cytotoxins which disrupt microtubule assembly and the second class of cytotoxins target DNA structure. Typically, cytotoxins will be coupled to the inventive antibody, antigen-binding fragment thereof, or to the heterodimeric immunoglobulin molecule as disclosed above via a linker. The term "linker" or "linker peptide" refers to a synthetic or artifical amino acid sequence that connects or links two molecules, such as e.g. two polypeptide sequences that link two polypeptide domains, or e.g. a protein and a cytostatic drug, or toxin. The term "synthetic" or "artifical" as used in the present invention refers to amino acid sequences that are not naturally occurring. For example, a linker which may covalently bound to the heterodimeric immunoglobulin molecule of the invention or the inventive antibody as disclosed above can be cleavable or non-cleavable. The term "cleavable" as used in the present invention refers to linkers which may be cleaved by proteases, acids, or by reduction of a disulfide body (e.g. glutathion-mediated or glutathion sensitive). For example, cleavable linkers may comprise valine-citrulline linkers, hydrazone linkers, or disulfide linkers.. Non-cleavable linkers which may e.g. be covalently bound to the amino donor-comprising substrate of the invention comprise maleimidocaproyl linker to MMAF (mc-MMAF), N-maleimidomethylcyclohexane-1-carboxylate (MCC), or mercapto-acetamidocaproyl linkers. For example, the linkers which are covalently coupled to the inventive heterodimeric bispecific immunoglobulin molecule may also include linkers as described in WO 2010/138719, or e.g. those described in WO 2014/093379.

Accordingly, cytotoxins which may e.g. be covalently bound to the linker according to the invention include doxorubicin, calicheamicin, auristatin, maytansine duoarmycin and analogs thereof, α-amaitin, tubulysin and analogs thereof. Methods for covalently attaching cytotoxins to linkers are known in the art and may e.g. be done according to the method disclosed in Mol. Pharmaceutics 2015, 12, 1813-1835. Cytotoxins may e.g. also be coupled to the inventive antibody comprising a human IgG moiety non-covalently through the use of Fab-anti-human Fc fragments conjugated to a cytotoxins via a non-cleavable linker. For example, commercial preparations of such Fab-anti-human Fc-cytotoxin conjugates may be used which comprise the cytotoxin α-amanitin (e.g. Fab-anti-human Fc-NC-AAMT manufactured by Moradec). The principles of using and assessing such antibody drug conjugates may e.g. be done as described in J Chromatogr B Analyt Technol Biomed Life Sci. 2016 May 24.

According to one embodiment the heterodimeric immunoglobulin molecule as disclosed above is afucosylated. The term "afucosylated" as used for the heterodimeric immunoglobulin molecule according to the invention refers to heterodimeric immunoglobulin molecule which are devoid of the sugar fucose, or which e.g. have only minor amounts of fusose in their N-glycan structure, e.g. less than 5%, 4%, 3%, 2% or less than 1% fucosylated N-glycans in any preparation of the heterodimeric immunoglobulin molecule according to the invention as disclosed above. In one aspect, the inventive antibody as disclosed above may be afucosylated. Typically, the *N*-glycan attached to Asn²⁹⁷ of human IgG1, e.g. of the inventive antibody as disclosed above, significantly enhances its binding to FcγRIIIa and thereby improves antibody-dependent cellular cytotoxicity (ADCC). Afucosylated heterodimeric immunoglobulin molecules according to the invention as disclosed above may e.g. be obtained by using cell lines for their production which are devoid of the GDP-fucose transporter SLC35C1, as described in Glycobiology. 2012;22:897-911, such as CHO-gmt5 cells. Alternatively, co-expression if GDP-6-deoxy-D-lyxo-hexulose reductase as described in Glycobiology. 2010 Dec;20(12):1607-18 may be used to manufacture afucosylated heterodimeric immunoglobulin molecules, or the inventive antibody and/or antigen-binding fragments thereof as disclosed above.

In one aspect the present disclosure provides for isolated polynucleotides which encode the inventive antibodies as disclosed abovecomprising the amino acid sequences according to SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, or any of their respective kinetic variants as disclosed above. The term "isolated" as used with the polynucleotides according to the disclosure refers to polynucleotides which are separated from e.g. constituents, cellular and otherwise, in which the polynucleotide are normally associated with in nature, e.g. the isolated polynucleotide is at least 80%, 90%, 95% pure by weight, i.e. devoid of contaminating constituents. For example, isolated polynucleotides of the disclosure may refer to a DNA molecule that is separated from sequences with which it is immediately contiguous (in the 5' and 3' directions) in the naturally occurring genome of the organism from which it was derived. For example, the "isolated polynucleotide" may comprise a DNA molecule inserted into a vector, such as a plasmid, expression plasmid or virus vector, or integrated into the genomic DNA of a procaryote or eucaryote. Accordingly, the present disclosure also provides for expression vectors which comprise at least one inventive polynucleotide. In one aspect the present disclosure also pertains to the use of the said polynucleotides in the manufacture of the inventive antibody or heterodimeric immunoglobulin molecule as disclosed above. In one aspect, the present disclosure also pertains to the manufacture of the inventive antibody or heterodimeric immunoglobulin molecule as disclosed above by means of expression in heterologous cell lines. For example, expression plasmids which may be used for expression of the inventive antibody, antigen-binding fragments thereof, or of the heterodimeric immunoglobulin molecule as disclosed above may e.g. comprise pCMV, pcDNA, p4X3, p4X4, p4X5, p4X6, pVL1392, pVL1393, pACYC177, PRS420, or if viral based vector systems are to be used e.g. pBABEpuro, pWPXL, pXP-derived vectors.may e.g. comprise pCMV, pcDNA, p4X3, p4X4, p4X5, p4X6, pVL1392, pVL1393, pACYC177, PRS420, or if viral based vector systems are to be used e.g. pBABEpuro, pWPXL, pXP-derived vectors.

In one aspect, the present disclosure provides for at least one host cell which comprises at least one inventive polynucleotide as disclosed above, e.g. a polynucleotide or vector or expression vector which encodes at least one of SEQ ID NO: 1, SEQ ID NO: 2, SEQ ID NO: 3, SEQ ID NO: 4, SEQ ID NO: 4, SEQ ID NO: 5, SEQ ID NO: 6, SEQ ID NO: 7, SEQ ID NO: 8 and its use in the manufacture of the inventive antibody or heterodimeric immunoglobulin molecule as disclosed above. For example, a host cell for use according to the disclosure may be a yeast cell, insect cell or mammalian cell. For example, the host cell of the disclosure may be an insect cell selected from Sf9, Sf21, S2, Hi5, or BTI-TN-5B1-4 cells, or e.g. the host cell of the disclosure may be a yeast cell selected from *Saccharomyces cerevisiae, Hansenula polymorpha, Schizosaccharomyces pombe, Schwanniomyces occidentalis, Kluyveromyceslactis, Yarrowia lipolytica* and *Pichia pastoris,* or e.g. the host cell of the disclosure may be a mammalian cell selected from HEK293, HEK293T, HEK293E, HEK 293F, NS0, per.C6, MCF-7, HeLa, Cos-1, Cos-7, PC-12, 3T3, Vero, vero-76, PC3, U87, SAOS-2, LNCAP, DU145, A431, A549, B35, H1299, HUVEC, Jurkat, MDA-MB-231, MDA-MB-468, MDA-MB-435, Caco-2, CHO, CHO-K1, CHO-B11, CHO-DG44, BHK, AGE1.HN, Namalwa, WI-38, MRC-5, HepG2, L-929, RAB-9, SIRC, RK13, 11B11, 1D3, 2.4G2, A-10, B-35, C-6, F4/80, IEC-18, L2, MH1C1, NRK, NRK-49F, NRK-52E, RMC, CV-1, BT, MDBK, CPAE, MDCK.1, MDCK.2, and D-17.

According to one embodiment the heterodimeric immunoglobulin molecule as disclosed above, or the inventive antibody as disclosed above may be used for the manufacture of a medicament for the treatment of cancer. For example, the inventive antibody heterdimeric immunoglobulin molecule coupled to a cytotoxin as disclosed above may be formulated into a pharmaceutical composition for administration to a patient in need thereof inflicted with cancer. A pharmaceutical composition according to the disclosure may e.g. comprise the heterodimeric immunoglobulin molecule of the invention coupled to a cytotoxin as disclosed above, or the antibody-drug conjugate as disclosed above (e.g. the inventive antibody coupled to a cytotoxin as disclosed above) in a concentration from about 10mg/ml, 20mg/ml, 25mg/ml, 30mg/ml, 35mg/ml, 40mg/ml, 45mg/ml, 50mg/ml, 55mg/ml, 60mg/ml to about 70mg/ml, 75mg/ml, 80mg/ml, 90mg/ml, 100mg/ml, 112mg/ml, 125mg/ml, 150mg/ml, 175mg/ml, 200mg/ml, or e.g. from about 10 mg/ml to about 20mg/ml, 25mg/ml, 30mg/ml, 35mg/ml, 40mg/ml, 45mg/ml, 50mg/ml, 55mg/ml, 60mg/ml to about 70mg/ml, 75mg/ml, 80mg/ml, 90mg/ml, 100mg/ml, 112mg/ml, 125mg/ml, 150mg/ml, 175mg/ml, 200mg/ml,or e.g. 20mg/ml, 25mg/ml, 30mg/ml, 35mg/ml, 40mg/ml, 45mg/ml, 50mg/ml, 55mg/ml, 60mg/ml to about 70mg/ml, 75mg/ml, 80mg/ml, 90mg/ml, 100mg/ml, 112mg/ml, 125mg/ml, 150mg/ml, 175mg/ml, 200mg/ml.

The pharmaceutical composition of the present disclosure may further comprise one or more pharmaceutically acceptable excipients. Pharmaceutically acceptable excipients for different dosage forms are well-known in the art and include carriers, diluents, fillers, binders, lubricants, disintegrants, glidants, colorants, pigments, taste masking agents, sweeteners, flavorants, plasticizers, and any acceptable auxiliary substances such as absorption enhancers, penetration enhancers, surfactants, co-surfactants, and specialized oils. Suited excipient(s) may e.g. be selected based on the dosage form, the intended mode of administration, the intended release rate, and manufacturing reliability. Examples of common types of excipients include various polymers, waxes, calcium phosphates, sugars, and the like.

In one aspect the disclosure also provides a method of treatment which comprises administering to a subject a therapeutically effective amount of the pharmaceutical composition as disclosed above. For example, the inventive method of treatment may comprise administering a person in need thereof inflicted with cancer from about 0.001 mg/kg to about 50 mg/kg of the inventive pharmaceutical composition, or from about 0.005 mg/kg to about 45 mg/kg, or from about 0.01 mg/kg to about 40 mg/kg, or from about 0.05 mg/kg to about 35 mg/kg, or from about 0.1 mg/kg, 0.5 mg/kg, 0.75 mg/kg, 1 mg/kg, 1.5 mg/kg, 2 mg/kg, 2.5 mg/kg, 3 mg/kg, 4 mg/kg, 5 mg/kg, 6 mg/kg 7 mg/kg, 8 mg/kg, 9 mg/kg, 10 mg/kg, 12.5 mg/kg, 15 mg/kg, 17.5 mg/kg, 20 mg/kg, 22.5 mg/kg, 25 mg/kg to about 26 mg kg/, 27 mg/kg, 28 mg/kg, 29 mg/kg, 30 mg/kg, 32.5 mg/kg, 35 mg/kg, 37.5 mg/kg, 40 mg/kg, 42.5 mg/kg, 45 mg/kg. As used the term "mg/kg" refers to mg of the inventive pharmaceutical composition/kg body weight in the present disclosure. For example, a pharmaceutically effective amount of the inventive pharmaceutical composition may be administered to an individual inflicted with cancer. The pharmaceutically effective amount depends on the individual, the type of cancer to be treated, the body weight and age of the individual, the level of the disease or the administration route, e.g. i.v., or subcutanteous. In one aspect, the present disclosure also provides a method of treating a patient inflicted with cancer with the inventive antibody as disclosed above, or with the inventive heterodimeric immunoglobulin molecule as disclosed above. For example, the method may comprise administering to a patient in need thereof (e.g. a cancer patient) a pharmaceutical composition comprising the inventive antibody in a concentration and dosing as disclosed above. For example, in one aspect the inventive pharmaceutical composition may be used in patients suffering from a tumor that expresses high levels of c-MET, or e.g. any of the c-MET variants disclosed herein. The term "high levels" as used in the inventive method of treatment refers to c-MET expression levels that are at least 2x, 5x, 10x, 15x, 20x, 25x, 50x higher than in a control tissue (e.g. tissue obtained form a healthy individual, or e.g. a cell line that does not express c-MET as assessed by qPCR, Western blotting, immunohistochemistry). For example, c-Met expression in the tumor of a patient may be assessed on tumor tissue which may be obtained by needle aspiration or surgical biopsy by means of immunohistochemistry, or circulating tumor DNA (ctDNA) in the patient's blood may be used to assess c-MET amplifications which correlate with high c-Met expression, as e.g. described in Mol Cancer Res. 2016 Jun;14(6):539-47. Obtaining the tumor tissue sample, or a blood sample from individuals inflicted with cancer does not form part of the present invention.

In one aspect, the inventive antibodies, antigen-binding fragments thereof, or the SEEDbodies as disclosed above may e.g. be used for diagnostic purposes to detect c-MET expression in a sample. The term "sample" as used in the present disclosure refers to tissue samples obtained or derived from tumor tissue or control tissue from a healthy donor, e.g. a human subject not inflicted with cancer. The sample may also be derived from non-human primates, or may be of mammalian origin, such as murine or rat origin. The term sample may also refer to single or individualized cells obtained from a tissue sample by e.g. means of a needle biopsy, whereby obtaining the sample from a human subject does not form part of this invention. The sample may be comprised of unfixed, viable cells, or may be comprised of fixed tissue or cells, such as formalin-fixed paraffin-embeded tissue or cells. The term sample according to the invention may for example also refer to cells from cancer cell lines, such as KP-4, U87MG, A549, NCI-H441, MKN-45, or EBC-1 and the like that are e.g. obtainable from ATCC. Detecting c-MET expression in a sample comprises contacting the sample with the inventive antibodies, antigen-binding fragments thereof, or SEEDbodies as disclosed above under conditions that allow specific binding to c-MET and subsequently detecting the inventive antibody, or antigen-binding fragment thereof, or the inventive SEEDbody as disclosed above, preferably by means of detecting the coupled detectable label as disclosed above.

### EXAMPLES

The following Examples are intended to further illustrate the invention.

### Example 1: HGF competition ELISA - displacement by inventive antibodies

Competition of recombinant human HGF with inventive antibodies binding to recombinant human c-MET ECD was detected by ELISA using HGF in solid phase. For this, 1.25 pmol HGF were immobilized on 96 well MaxiSorp® plates overnight at 4°C. After blocking plates with 2% BSA in PBS-T, 1.13 pmol biotinylated c-MET ECD pre-incubated with serial dilutions of antibodies (0.2-200 nM) were added to plates. Binding was revealed using HRP-conjugated streptavidin followed by addition of 1 step Ultra TMB ELISA substrate solution and sulfuric acid. Resulting absorbance for c-MET ECD binding to HGF without addition of anti-c-MET directed antibody was defined as 100% HGF binding. Anti-HEL SEED was used as an unrelated isotype control antibody. Data were plotted as % HGF binding against the logarithm of the antibody concentration and fitted to a sigmoidal dose-response curve with variable slope (4PL) using GraphPad Prism 5®.

### Example 2: Receptor phosphorylation assay - inhibition of c-MET signaling

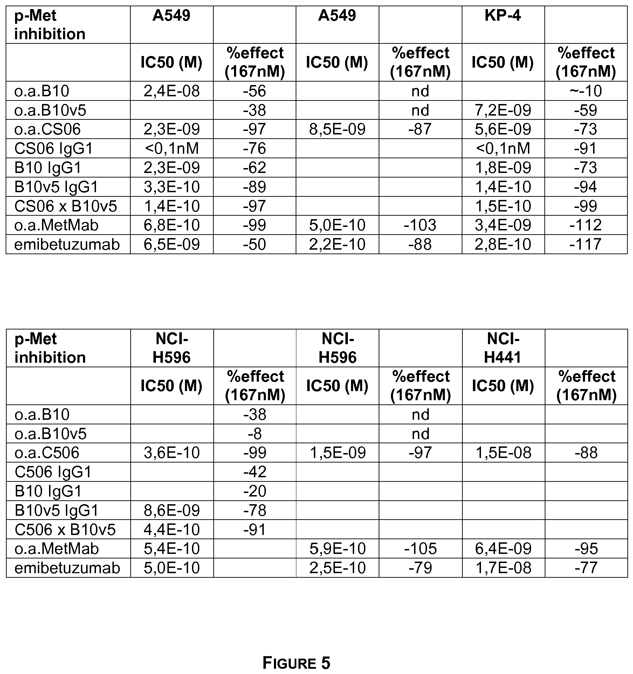

To assess the effect of binding of the inventive antibody and inventive heterodimeric immunoglobulin molecule on c-MET- mediated signaling phosphorylation levels c-MET were determined by c-MET capture electrochemiluminescence (ECL) ELISA (MSD assay). All reagents were obtained from Meso Scale Discovery and prepared according to the manufacturer's instructions. Briefly, cells were plated in 96-well tissue culture plates (Sigma-Aldrich) one day before treatment, serum starved and treated with serially diluted antibodies (0 - 167 nM in starvation medium) for 1 h at 37°C, 5% CO₂. Upon stimulation with either 100 ng/ml HGF (R&D Systems) for 5 min at 37°C, cells were lysed with ice-cold lysis buffer supplemented with protease and phosphatase inhibitors (Calbiochem). High bind 96-well plates including electrodes (Meso Scale Discovery) were coated with capture anti-total c-MET (Cell Signaling Technologies) antibody (Abcam) followed by blocking with 3% Block A in PBS supplemented with 0.05% Tween®20. After incubation with cell lysates, detection was carried out with anti-phospho c-MET (Cell Signaling Technologies), anti-phospho-tyrosine antibodies (R&D Systems) and by the supplier recommended detection substances. Measurements were performed with the SECTOR® Imager 6000 (Meso Scale Discovery). For quantification of phospho-AKT levels, the Phospho(Ser473)/Total AKT Assay Whole Cell Lyate Kit (Meso Scale Discovery) was used. Dose response curves were plotted as the logarithm of antibody concentration versus ECL signal. IC₅₀ values were calculated by a 3PL fitting model using GraphPad Prism 5 (GraphPad Software, Inc.), see e.g. data provided in Figure 5.

### Example 3: Epitope Binning of c-MET binders using Bio-Layer Interferometry (BLI)

An epitope binning experiment was carried out with c-MET antibodies which were used in the bispecific antibodies and compared to reference antibodies from the literature (MetMAb, Emibetuzumab, h224G11). Biosensor experiments using bio-layer interferometry were performed on an Octet Red platform (Forte Bio) equipped with anti-human Fc (AHC) biosensor tips (Forte Bio). All data were collected at 30 °C in kinetics buffer (PBS pH 7.4, 0.1% BSA, 0.02% Tween-20. Human c-MET ECD-His (HGFR, hepatocyte growth factor receptor extracellular domain) was produced and purified in-house. Biosensor tips were equilibrated 30 sec in PBS. Then, 25 nM for bivalent IgGs and 50 nM for monovalent one-armed antibodies in PBS were immobilized on biosensor tips for 200 sec as primary antibody. Tips were quenched with 400 nM of a non-related control antibody (anti-hen egg lysozyme, anti-HEL SEED, diluted in PBS) to minimize subsequent binding of secondary antibodies to biosensor tips. Following acquisition of a baseline in kinetics buffer for 60 sec, human c-MET-ECD was subjected to immobilized primary antibodies for 600 sec. Afterwards, interactions of secondary anti-c-MET antibodies to c-MET-ECD bound to immobilized primary antibodies was analyzed for 600 sec. Analysis of secondary antibody binding was analyzed visually by distinguishing simultaneous binding characterized by a higher binding rate [nM] compared to a non-related isotype control (anti-HEL SEED).

### Example 4: Cytotoxicity assays

To asses cytotoxicity of the inventive antibody drug conjugates which were non-covalent conjugates of the cytotoxin to the Fc portion of the inventive antibody via anti-human Fc-Fab toxin conjugates (MORADEC, catalog number AH205-AM).

Cell viability was quantified using the CellTiter-Glo® assay (Promega) and was performed according to the manufacturer's instructions. Briefly, cells were detached and seeded in the inner wells of opaque white tissue culture treated 96 well plates. The seeding cell number ranged from 8,000 to 15,000 viable cells per well depending on the cell line in 80 µl cell line specific medium. Cells were allowed to attach at least 3 h in a humidified chamber at 37°C, 5% CO₂ before ADC treatment (ranging from 50 to 0.01 nM final) in duplicates in cell line specific medium. After 72 h, viability of cells was detected by adding 100 µl per well of CellTiter-Glo® reagent with subsequent mixing on a plate shaker for 2 min at 350 rpm and 10 min incubation in the dark at RT. Luminescence was measured at a Synergy 4 plate reader (chapter 3.9 and 3.10) with a read time of 0.5 seconds per well (sensitivity: 170). Background luminescence in wells with only medium plus the CellTiter-Glo® reagent was subtracted. Data were plotted as percentage of untreated cell viability versus the logarithm of antibody concentration and fitted with 3PL model using GraphPad Prism 5 (chapter 3.10). Data from at least three independent experiments with duplicates were used to calculate mean IC₅₀.

### SEQUENCE LISTING

<110> Merck Patent GmbH
<120> Anti-c-MET antibodies and antibody drug conjugates thereof for
   efficient tumor inihibtion
<130> P16/183
<160> 11
<170> BiSSAP 1.3.6
<210> 1
   <211> 116
   <212> PRT
   <213> Artificial Sequence
<220>
   <223> B10 light chain sequence, which may include one or more, or all of the following mutations (in IMGT numbering): V3A, T11V, T14S, R18S, R43Q, L45P, E74D, T85N, S86T, A90T, T92S G100A
<400> 1
<210> 2
   <211> 117
   <212> PRT
   <213> Artificial Sequence
<220>
   <223> B10 heavy chain amino acid sequence, which may include the mutations (in IMGT numbering): Q6E
<400> 2
<210> 3
   <211> 115
   <212> PRT
   <213> Artificial Sequence
<220>
   <223> F06 light chain sequence may comprise one or more, or all of the mutations (in IMGT numbering): Q1S, L2Y, S7P, K44Q, I51V, V71I,
<400> 3
<210> 4
   <211> 124
   <212> PRT
   <213> Artificial Sequence
<220>
   <223> F06 heavy chain sequence which may comprise one or more, or all of the mutations (in IGMT numbering) Q5V, A19V, M115I, M115L, M115V, M115A, M115F
<400> 4
<210> 5
   <211> 114
   <212> PRT
   <213> Artificial Sequence
<220>
   <223> B10v5 light chain sequence which may comprise one or more, or all of the mutations (in IMGT numbering) E1S, P2Y, E17Q, T20R, P22T, R45K, L51V, T85N, S93R, F103Y
<400> 5
<210> 6
   <211> 117
   <212> PRT
   <213> Artificial Sequence
<220>
   <223> B10v5 heavy chain sequence
<400> 6
<210> 7
   <211> 114
   <212> PRT
   <213> Artificial Sequence
<220>
   <223> CS06 light chain sequence
<400> 7
<210> 8
   <211> 124
   <212> PRT
   <213> Artificial Sequence
<220>
   <223> CS06 heavy chain sequence which may comprise one or more, or all of the mutations (in IMGT numbering) Q3R, Y37N, N66I, Q110S, D111.1Y, Y11.2D, S126Y
<400> 8
<210> 9
   <211> 106
   <212> PRT
   <213> Artificial Sequence
<220>
   <223> AG-SEED
<400> 9
<210> 10
   <211> 106
   <212> PRT
   <213> Artificial Sequence
<220>
   <223> GA-SEED
<400> 10
<210> 11
   <211> 1390
   <212> PRT
   <213> Homo sapiens
<220>
   <223> MET_HUMAN Hepatocyte growth factor receptor, UniProt No. P08581
<400> 11
   Met Lys Ala Pro Ala Val Leu Ala Pro Gly Ile Leu Val Leu Leu Phe
   1               5                   10                  15
   Thr Leu Val Gln Arg Ser Asn Gly Glu Cys Lys Glu Ala Leu Ala Lys
               20                  25                  30
   Ser Glu Met Asn Val Asn Met Lys Tyr Gln Leu Pro Asn Phe Thr Ala
           35                  40                  45
   Glu Thr Pro Ile Gln Asn Val Ile Leu His Glu His His Ile Phe Leu
       50                  55                  60
   Gly Ala Thr Asn Tyr Ile Tyr Val Leu Asn Glu Glu Asp Leu Gln Lys
   65                  70                  75                  80
   Val Ala Glu Tyr Lys Thr Gly Pro Val Leu Glu His Pro Asp Cys Phe
                   85                  90                  95
   Pro Cys Gln Asp Cys Ser Ser Lys Ala Asn Leu Ser Gly Gly Val Trp
               100                 105                 110
   Lys Asp Asn Ile Asn Met Ala Leu Val Val Asp Thr Tyr Tyr Asp Asp
           115                 120                 125
   Gln Leu Ile Ser Cys Gly Ser Val Asn Arg Gly Thr Cys Gln Arg His
       130                 135                 140
   Val Phe Pro His Asn His Thr Ala Asp Ile Gln Ser Glu Val His Cys
   145                 150                 155                 160
   Ile Phe Ser Pro Gln Ile Glu Glu Pro Ser Gln Cys Pro Asp Cys Val
                   165                 170                 175
   Val Ser Ala Leu Gly Ala Lys Val Leu Ser Ser Val Lys Asp Arg Phe
               180                 185                 190
   Ile Asn Phe Phe Val Gly Asn Thr Ile Asn Ser Ser Tyr Phe Pro Asp
           195                 200                 205
   His Pro Leu His Ser Ile Ser Val Arg Arg Leu Lys Glu Thr Lys Asp
       210                 215                 220
   Gly Phe Met Phe Leu Thr Asp Gln Ser Tyr Ile Asp Val Leu Pro Glu
   225                 230                 235                 240
   Phe Arg Asp Ser Tyr Pro Ile Lys Tyr Val His Ala Phe Glu Ser Asn
                   245                 250                 255
   Asn Phe Ile Tyr Phe Leu Thr Val Gln Arg Glu Thr Leu Asp Ala Gln
               260                 265                 270
   Thr Phe His Thr Arg Ile Ile Arg Phe Cys Ser Ile Asn Ser Gly Leu
           275                 280                 285
   His Ser Tyr Met Glu Met Pro Leu Glu Cys Ile Leu Thr Glu Lys Arg
       290                 295                 300
   Lys Lys Arg Ser Thr Lys Lys Glu Val Phe Asn Ile Leu Gln Ala Ala
   305                 310                 315                 320
   Tyr Val Ser Lys Pro Gly Ala Gln Leu Ala Arg Gln Ile Gly Ala Ser
                   325                 330                 335
   Leu Asn Asp Asp Ile Leu Phe Gly Val Phe Ala Gln Ser Lys Pro Asp
               340                 345                 350
   Ser Ala Glu Pro Met Asp Arg Ser Ala Met Cys Ala Phe Pro Ile Lys
           355                 360                 365
   Tyr Val Asn Asp Phe Phe Asn Lys Ile Val Asn Lys Asn Asn Val Arg
       370                 375                 380
   Cys Leu Gln His Phe Tyr Gly Pro Asn His Glu His Cys Phe Asn Arg
   385                 390                 395                 400
   Thr Leu Leu Arg Asn Ser Ser Gly Cys Glu Ala Arg Arg Asp Glu Tyr
                   405                 410                 415
   Arg Thr Glu Phe Thr Thr Ala Leu Gln Arg Val Asp Leu Phe Met Gly
               420                 425                 430
   Gln Phe Ser Glu Val Leu Leu Thr Ser Ile Ser Thr Phe Ile Lys Gly
           435                 440                 445
   Asp Leu Thr Ile Ala Asn Leu Gly Thr Ser Glu Gly Arg Phe Met Gln
       450                 455                 460
   Val Val Val Ser Arg Ser Gly Pro Ser Thr Pro His Val Asn Phe Leu
   465                 470                 475                 480
   Leu Asp Ser His Pro Val Ser Pro Glu Val Ile Val Glu His Thr Leu
                   485                 490                 495
   Asn Gln Asn Gly Tyr Thr Leu Val Ile Thr Gly Lys Lys Ile Thr Lys
               500                 505                 510
   Ile Pro Leu Asn Gly Leu Gly Cys Arg His Phe Gln Ser Cys Ser Gln
           515                 520                 525
   Cys Leu Ser Ala Pro Pro Phe Val Gln Cys Gly Trp Cys His Asp Lys
       530                 535                 540
   Cys Val Arg Ser Glu Glu Cys Leu Ser Gly Thr Trp Thr Gln Gln Ile
   545                 550                 555                 560
   Cys Leu Pro Ala Ile Tyr Lys Val Phe Pro Asn Ser Ala Pro Leu Glu
                   565                 570                 575
   Gly Gly Thr Arg Leu Thr Ile Cys Gly Trp Asp Phe Gly Phe Arg Arg
               580                 585                 590
   Asn Asn Lys Phe Asp Leu Lys Lys Thr Arg Val Leu Leu Gly Asn Glu
           595                 600                 605
   Ser Cys Thr Leu Thr Leu Ser Glu Ser Thr Met Asn Thr Leu Lys Cys
       610                 615                 620
   Thr Val Gly Pro Ala Met Asn Lys His Phe Asn Met Ser Ile Ile Ile
   625                 630                 635                 640
   Ser Asn Gly His Gly Thr Thr Gln Tyr Ser Thr Phe Ser Tyr Val Asp
                   645                 650                 655
   Pro Val Ile Thr Ser Ile Ser Pro Lys Tyr Gly Pro Met Ala Gly Gly
               660                 665                 670
   Thr Leu Leu Thr Leu Thr Gly Asn Tyr Leu Asn Ser Gly Asn Ser Arg
           675                 680                 685
   His Ile Ser Ile Gly Gly Lys Thr Cys Thr Leu Lys Ser Val Ser Asn
       690                 695                 700
   Ser Ile Leu Glu Cys Tyr Thr Pro Ala Gln Thr Ile Ser Thr Glu Phe
   705                 710                 715                 720
   Ala Val Lys Leu Lys Ile Asp Leu Ala Asn Arg Glu Thr Ser Ile Phe
                   725                 730                 735
   Ser Tyr Arg Glu Asp Pro Ile Val Tyr Glu Ile His Pro Thr Lys Ser
               740                 745                 750
   Phe Ile Ser Gly Gly Ser Thr Ile Thr Gly Val Gly Lys Asn Leu Asn
           755                 760                 765
   Ser Val Ser Val Pro Arg Met Val Ile Asn Val His Glu Ala Gly Arg
       770                 775                 780
   Asn Phe Thr Val Ala Cys Gln His Arg Ser Asn Ser Glu Ile Ile Cys
   785                 790                 795                 800
   Cys Thr Thr Pro Ser Leu Gln Gln Leu Asn Leu Gln Leu Pro Leu Lys
                   805                 810                 815
   Thr Lys Ala Phe Phe Met Leu Asp Gly Ile Leu Ser Lys Tyr Phe Asp
               820                 825                 830
   Leu Ile Tyr Val His Asn Pro Val Phe Lys Pro Phe Glu Lys Pro Val
           835                 840                 845
   Met Ile Ser Met Gly Asn Glu Asn Val Leu Glu Ile Lys Gly Asn Asp
       850                 855                 860
   Ile Asp Pro Glu Ala Val Lys Gly Glu Val Leu Lys Val Gly Asn Lys
   865                 870                 875                 880
   Ser Cys Glu Asn Ile His Leu His Ser Glu Ala Val Leu Cys Thr Val
                   885                 890                 895
   Pro Asn Asp Leu Leu Lys Leu Asn Ser Glu Leu Asn Ile Glu Trp Lys
               900                 905                 910
   Gln Ala Ile Ser Ser Thr Val Leu Gly Lys Val Ile Val Gln Pro Asp
           915                 920                 925
   Gln Asn Phe Thr Gly Leu Ile Ala Gly Val Val Ser Ile Ser Thr Ala
       930                 935                 940
   Leu Leu Leu Leu Leu Gly Phe Phe Leu Trp Leu Lys Lys Arg Lys Gln
   945                 950                 955                 960
   Ile Lys Asp Leu Gly Ser Glu Leu Val Arg Tyr Asp Ala Arg Val His
                   965                 970                 975
   Thr Pro His Leu Asp Arg Leu Val Ser Ala Arg Ser Val Ser Pro Thr
               980                 985                 990
   Thr Glu Met Val Ser Asn Glu Ser Val Asp Tyr Arg Ala Thr Phe Pro
           995                 1000                1005
   Glu Asp Gln Phe Pro Asn Ser Ser Gln Asn Gly Ser Cys Arg Gln Val
       1010                1015                1020
   Gln Tyr Pro Leu Thr Asp Met Ser Pro Ile Leu Thr Ser Gly Asp Ser
   1025                1030                1035                1040
   Asp Ile Ser Ser Pro Leu Leu Gln Asn Thr Val His Ile Asp Leu Ser
                   1045                1050                1055
   Ala Leu Asn Pro Glu Leu Val Gln Ala Val Gln His Val Val Ile Gly
               1060                1065                1070
   Pro Ser Ser Leu Ile Val His Phe Asn Glu Val Ile Gly Arg Gly His
           1075                1080                1085
   Phe Gly Cys Val Tyr His Gly Thr Leu Leu Asp Asn Asp Gly Lys Lys
       1090                1095                1100
   Ile His Cys Ala Val Lys Ser Leu Asn Arg Ile Thr Asp Ile Gly Glu
   1105                1110                1115                1120
   Val Ser Gln Phe Leu Thr Glu Gly Ile Ile Met Lys Asp Phe Ser His
                   1125                1130                1135
   Pro Asn Val Leu Ser Leu Leu Gly Ile Cys Leu Arg Ser Glu Gly Ser
               1140                1145                1150
   Pro Leu Val Val Leu Pro Tyr Met Lys His Gly Asp Leu Arg Asn Phe
           1155                1160                1165
   Ile Arg Asn Glu Thr His Asn Pro Thr Val Lys Asp Leu Ile Gly Phe
       1170                1175                1180
   Gly Leu Gln Val Ala Lys Gly Met Lys Tyr Leu Ala Ser Lys Lys Phe
   1185                1190                1195                1200
   Val His Arg Asp Leu Ala Ala Arg Asn Cys Met Leu Asp Glu Lys Phe
                   1205                1210                1215
   Thr Val Lys Val Ala Asp Phe Gly Leu Ala Arg Asp Met Tyr Asp Lys
               1220                1225                1230
   Glu Tyr Tyr Ser Val His Asn Lys Thr Gly Ala Lys Leu Pro Val Lys
           1235                1240                1245
   Trp Met Ala Leu Glu Ser Leu Gln Thr Gln Lys Phe Thr Thr Lys Ser
       1250                1255                1260
   Asp Val Trp Ser Phe Gly Val Leu Leu Trp Glu Leu Met Thr Arg Gly
   1265                1270                1275                1280
   Ala Pro Pro Tyr Pro Asp Val Asn Thr Phe Asp Ile Thr Val Tyr Leu
                   1285                1290                1295
   Leu Gln Gly Arg Arg Leu Leu Gln Pro Glu Tyr Cys Pro Asp Pro Leu
               1300                1305                1310
   Tyr Glu Val Met Leu Lys Cys Trp His Pro Lys Ala Glu Met Arg Pro
           1315                1320                1325
   Ser Phe Ser Glu Leu Val Ser Arg Ile Ser Ala Ile Phe Ser Thr Phe
       1330                1335                1340
   Ile Gly Glu His Tyr Val His Val Asn Ala Thr Tyr Val Asn Val Lys
   1345                1350                1355                1360
   Cys Val Ala Pro Tyr Pro Ser Leu Leu Ser Ser Glu Asp Asn Ala Asp
                   1365                1370                1375
   Asp Glu Val Asp Thr Arg Pro Ala Ser Phe Trp Glu Thr Ser
               1380                1385                1390

## Claims

1. Anti-c-Met antibody or antigen-binding fragment thereof, wherein the antibody or antigen-binding fragment thereof binds to human c-MET with an affinity of at least 10⁻⁸M, wherein the antibody is an IgG type antibody and wherein the antibody comprises heavy and light chain amino acid sequences according to SEQ ID NO:1 and SEQ ID NO: 2, or SEQ ID NO: 3 and SEQ ID NO: 4, or SEQ ID NO: 5 and SEQ ID NO: 6, or SEQ ID NO: 7 and SEQ ID NO: 8.

2. Antibody or antigen-binding fragment thereof according to claim 1, wherein the antibody or antigen-binding fragment thereof binds to human c-MET variant N375S.

3. Antibody or antigen-binding fragment therof according to claim 1 or claim 2, wherein the antibody or antigen-binding fragment thereof binds to an epitope comprised in the SEMA domain of human c-MET and inhibits c-MET signaling.

4. Antibody or antigen-binding fragment thereof according to any one of claims 1-3, wherein the antibody or antigen-binding fragment thereof binds to an epitope comprised in IPT domains 1-4 of human c-MET and inhibits c-MET signaling.

5. Antibody or antigen-binding fragment thereof according to claim 3 or claim 4, wherein the antibody or antigen-binding fragment thereof inhibits binding of recombinant human HGF recombinant to human c-MET ECD at a concemtration of 0.9x10⁻⁹M or less by 50% in an enzyme-linked immunosorbent assay using HGF in solid phase.

6. Antibody or antigen-binding fragment thereof according to any one of claims 1-5, wherein the antibody or antigen-binding fragment is a Fab.

7. Antibody or antigen-binding fragment thereof according to any one of claims 1-5, wherein the antibody or antigen-binding fragment is a F(ab')₂.

8. Antibody or antigen-binding fragment thereof according to any one of claims 1-5, wherein the antibody or antigen-binding fragment is a scFv.

9. Antibody or antigen-binding fragment thereof according to any one of claims 1-8, wherein the antibody or antigen-binding fragment thereof is further coupled to a diagnostic or therapeutic agent.

10. Heterodimeric immunoglobulin molecule comprising
(i) a first and second Fab or scFv fragment which specifically bind to human c-MET, and
(ii) an antibody hinge region, an antibody CH2 domain and an antibody CH3 domain comprising a hybrid protein-protein interaction interface domain wherein said interaction interface domain is formed by amino acid segments of the CH3 domain of a first member and amino acid segments of the CH3 domain of said second member, wherein said protein-protein interface domain of the first chain is interacting with the protein-protein-interface of the second chain by homodimerization of the corresponding amino acid segments of the same member of the immunoglobulin superfamily within said interaction domains,
wherein the first engineered immunoglobulin chain has the polypeptide sequence ("AG-SEED"):
GQPFRPEVHLLPPSREEMTKNQVSLTCLARGFYPKDIAVEWESNGQPENNYKTTP SRQEPSQGTT TFAVTSKLTVDKSRWQQGNVFSCSVMHEALHNHYTQKTISL and the second engineered immunoglobulin chain has the polypeptide sequence ("GA-SEED"):
GQPREPQVYTLPPPSEELALNELVTLTCLVKGFYPSDIAVEWLQGSQELPREKYLT WAPVLDSDG SFFLYSILRVAAEDWKKGDTFSCSVMHEALHNHYTQKSLDR and wherein the first and/or second Fab or scFv fragment comprise at least two of the amino acid sequences according to SEQ ID NO: 1, SEQ IDNO: 2, SEQ ID NO: 3, SEQ ID NO: 4, SEQ ID NO: 5, SEQ ID NO: 6, SEQ ID NO: 7, SEQ ID NO: 8.

11. Heterodimeric immunoglobulin molecule according to claim 10, whereby the heterodimeric immunoglobulin molecule is further coupled to a diagnostic or therapeutic agent.

12. Antibody according to claim 9, or the heterodimeric immunoglobulin molecule according to claim 11, wherein the therapeutic agent is a cytotoxin.

13. Heterodimeric immunoglobulin molecule according to claim 12, wherein the heterodimeric immunoglobulin molecule is afucosylated.

14. Antibody according to claim 12, or the heterdimeric immunoglobulin molecule according to claim 12 or claim 13 for use in the treatment of cancer.

## Patentansprüche

1. Anti-c-Met-Antikörper oder antigenbindendes Fragment davon, wobei der Antikörper oder das antigenbindende Fragment davon mit einer Affinität von mindestens 10⁻⁸ M an humanes c-MET bindet, wobei der Antikörper ein Antikörper vom IgG-Typ ist und wobei der Antikörper Aminosäuresequenzen der schweren und leichten Kette gemäß SEQ ID NO: 1 und SEQ ID NO: 2, oder SEQ ID NO: 3 und SEQ ID NO: 4, oder SEQ ID NO: 5 und SEQ ID NR. 6, oder SEQ ID NO: 7 und SEQ ID NO: 8 enthält.

2. Antikörper oder antigenbindendes Fragment davon nach Anspruch 1, wobei der Antikörper oder das antigenbindende Fragment davon an die humane c-MET-Variante N375S bindet.

3. Antikörper oder antigenbindendes Fragment davon nach Anspruch 1 oder Anspruch 2, wobei der Antikörper oder das antigenbindende Fragment davon an ein Epitop bindet, das in der SEMA-Domäne von humanem c-MET enthalten ist, und die c-MET-Signalübertragung hemmt.

4. Antikörper oder antigenbindendes Fragment davon nach einem der Ansprüche 1 bis 3, wobei der Antikörper oder das antigenbindende Fragment davon an ein Epitop bindet, das in den IPT-Domänen 1 bis 4 von humanem c-MET enthalten ist, und die c-MET-Signalübertragung hemmt.

5. Antikörper oder antigenbindendes Fragment davon nach Anspruch 3 oder Anspruch 4, wobei der Antikörper oder das antigenbindende Fragment davon die Bindung von rekombinantem humanem HGF, der gegenüber humanem c-MET ECD rekombinant ist, bei einer Konzentration von 0,9 x 10⁻⁹ M oder weniger in einem enzymgebundenen Immunosorbens-Assay unter Verwendung von HGF in fester Phase um 50% hemmt.

6. Antikörper oder antigenbindendes Fragment davon nach einem der Ansprüche 1 bis 5, wobei der Antikörper oder das antigenbindende Fragment ein Fab ist.

7. Antikörper oder antigenbindendes Fragment davon nach einem der Ansprüche 1 bis 5, wobei der Antikörper oder das antigenbindende Fragment ein F(ab')₂ ist.

8. Antikörper oder antigenbindendes Fragment davon nach einem der Ansprüche 1 bis 5, wobei der Antikörper oder das antigenbindende Fragment ein scFv ist.

9. Antikörper oder antigenbindendes Fragment davon nach einem der Ansprüche 1 bis 8, wobei der Antikörper oder das antigenbindende Fragment davon zudem an ein diagnostisches oder therapeutisches Mittel gekoppelt ist.

10. Heterodimeres Immunglobulinmolekül, enthaltend
(i) ein erstes und zweites Fab- oder scFv-Fragment, die spezifisch an humanes c-MET binden, und
(ii) eine Antikörper-Gelenkregion, eine Antikörper-CH2-Domäne und eine Antikörper-CH3-Domäne, enthaltend eine Hybrid-Protein-Protein-Wechselwirkungsschnittstellendomäne, wobei die Wechselwirkungsschnittstellendomäne durch Aminosäuresegmente der CH3-Domäne eines ersten Mitglieds und Aminosäuresegmente der CH3-Domäne des zweiten Mitglieds gebildet wird, wobei die Protein-Protein-Schnittstellendomäne der ersten Kette mit der Protein-Protein-Schnittstelle der zweiten Kette durch Homodimerisierung der entsprechenden Aminosäuresegmente desselben Mitglieds der Immunglobulin-Superfamilie innerhalb der Wechselwirkungsdomänen interagiert,
wobei die erste gentechnisch erzeugte Immunglobulinkette die Polypeptidsequenz ("AG-SEED") aufweist:
GQPFRPEVHLLPPSREEMTKNQVSLTCLARGFYPKDIAVEWESNGQPENNYKTTP SRQEPSQGTT TFAVTSKLTVDKSRWQQGNVFSCSVMHEALHNHYTQKTISL, und die zweite gentechnisch erzeugte Immunglobulinkette die Polypeptidsequenz ("GA-SEED") aufweist:
GQPREPQVYTLPPPSEELALNELVTLTCLVKGFYPSDIAVEWLQGSQELPREKYLT WAPVLDSDG SFFLYSILRVAAEDWKKGDTFSCSVMHEALHNHYTQKSLDR, und wobei das erste und/oder zweite Fab- oder scFv-Fragment mindestens zwei der Aminosäuresequenzen gemäß SEQ ID NO: 1, SEQ IDNO: 2, SEQ ID NO: 3, SEQ ID NO: 4, SEQ ID NO: 5, SEQ ID NO: 6, SEQ ID NO: 7, SEQ ID NO: 8 enthält.

11. Heterodimeres Immunglobulinmolekül nach Anspruch 10, wobei das heterodimere Immunglobulinmolekül zudem an ein diagnostisches oder therapeutisches Mittel gekoppelt ist.

12. Antikörper nach Anspruch 9 oder heterodimeres Immunglobulinmolekül nach Anspruch 11, wobei das therapeutische Mittel ein Cytotoxin ist.

13. Heterodimeres Immunglobulinmolekül nach Anspruch 12, wobei das heterodimere Immunglobulinmolekül afucosyliert ist.

14. Antikörper nach Anspruch 12 oder heterdimeres Immunglobulinmolekül nach Anspruch 12 oder Anspruch 13 zur Verwendung bei der Behandlung von Krebs.

## Revendications

1. Anticorps anti-c-Met ou fragment de liaison à l'antigène de celui-ci, l'anticorps ou le fragment de liaison à l'antigène de celui-ci se fixant à c-MET humain avec une affinité d'au moins 10⁻⁸ M, où l'anticorps est un anticorps de type IgG et où l'anticorps comprend des séquences d'acides aminés de chaînes lourdes et légères selon SEQ ID NO:1 et SEQ ID NO: 2, ou SEQ ID NO: 3 et SEQ ID NO: 4, ou SEQ ID NO: 5 et SEQ ID NO: 6, ou SEQ ID NO: 7 et SEQ ID NO: 8.

2. Anticorps ou fragment de liaison à l'antigène de celui-ci selon la revendication 1, l'anticorps ou le fragment de liaison à l'antigène de celui-ci se fixant au variant N375S de c-MET humain.

3. Anticorps ou fragment de liaison à l'antigène de celui-ci selon la revendication 1 ou la revendication 2, l'anticorps ou le fragment de liaison à l'antigène de celui-ci se fixant à un épitope compris dans le domaine SEMA de c-MET humain et inhibant la signalisation de c-MET.

4. Anticorps ou fragment de liaison à l'antigène de celui-ci selon l'une quelconque des revendications 1-3, l'anticorps ou le fragment de liaison à l'antigène de celui-ci se fixant à un épitope compris dans les domaines IPT 1-4 de c-MET humain et inhibant la signalisation de c-MET.

5. Anticorps ou fragment de liaison à l'antigène de celui-ci selon la revendication 3 ou la revendication 4, l'anticorps ou le fragment de liaison à l'antigène de celui-ci inhibant la fixation de HGF humain recombiné à l'ECD de c-MET humain selon une concentration de 0,9×10⁻⁹ M ou moins, de 50% dans une méthode immunoenzymatique utilisant du HGF en phase solide.

6. Anticorps ou fragment de liaison à l'antigène de celui-ci selon l'une quelconque des revendications 1-5, l'anticorps ou le fragment de liaison à l'antigène étant un Fab.

7. Anticorps ou fragment de liaison à l'antigène de celui-ci selon l'une quelconque des revendications 1-5, l'anticorps ou le fragment de liaison à l'antigène étant un F(ab')₂.

8. Anticorps ou fragment de liaison à l'antigène de celui-ci selon l'une quelconque des revendications 1-5, l'anticorps ou le fragment de liaison à l'antigène étant un scFv.

9. Anticorps ou fragment de liaison à l'antigène de celui-ci selon l'une quelconque des revendications 1-8, l'anticorps ou le fragment de liaison à l'antigène de celui-ci étant en outre couplé à un agent de diagnostic ou thérapeutique.

10. Molécule d'immunoglobuline hétérodimérique, comprenant
(i) un premier et un deuxième fragment Fab ou scFv qui se fixe spécifiquement à c-MET humain, et
(ii) une région charnière d'anticorps, un domaine CH2 d'anticorps et un domaine CH3 d'anticorps comprenant un domaine d'interface d'interaction protéine-protéine hybride, où ledit domaine d'interface d'interaction est formé par des segments d'acides aminés du domaine CH3 d'un premier membre et des segments d'acides aminés du domaine CH3 dudit deuxième membre, où ledit domaine d'interface protéine-protéine de la première chaîne interagit avec l'interface protéine-protéine de la deuxième chaîne par homodimérisation des segments d'acides aminés correspondants du même membre de la superfamille d'immunoglobulines au sein desdits domaines d'interaction,
où la première chaîne d'immunoglobuline ingéniérisée possède la séquence polypeptidique (« AG-SEED ») :
GQPFRPEVHLLPPSREEMTKNQVSLTCLARGFYPKDIAVEWESNGQPENNYKTTP SRQEPSQGTT TFAVTSKLTVDKSRWQQGNVFSCSVMHEALHNHYTQKTISL et la deuxième chaîne d'immunoglobuline ingéniérisée possède la séquence polypeptidique (« GA-SEED ») :
GQPREPQVYTLPPPSEELALNELVTLTCLVKGFYPSDIAVEWLQGSQELPREKYLT WAPVLDSDG SFFLYSILRVAAEDWKKGDTFSCSVMHEALHNHYTQKSLDR et
où le premier et/ou deuxième fragment Fab ou scFv comprend au moins deux parmi les séquences d'acides aminés selon SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8.

11. Molécule d'immunoglobuline hétérodimérique selon la revendication 10, la molécule d'immunoglobuline hétérodimérique étant en outre couplée à un agent de diagnostic ou thérapeutique.

12. Anticorps selon la revendication 9, ou molécule d'immunoglobuline hétérodimérique selon la revendication 11, l'agent thérapeutique étant une cytotoxine.

13. Molécule d'immunoglobuline hétérodimérique selon la revendication 12, la molécule d'immunoglobuline hétérodimérique étant afucosylée.

14. Anticorps selon la revendication 12, ou molécule d'immunoglobuline hétérodimérique selon la revendication 12 ou la revendication 13, pour une utilisation dans le traitement du cancer.
